(19) Europäisches Patentamt
European Patent Office
Office européen des brevets

(11) EP 2 695 901 B1

(12) **EUROPÄISCHE PATENTSCHRIFT**

(45) Veröffentlichungstag und Bekanntmachung des Hinweises auf die Patenterteilung:
**14.09.2016 Patentblatt 2016/37**

(51) Int Cl.:
***C08G 18/77*** *(2006.01)* ***C08G 63/16*** *(2006.01)*
***C08G 69/26*** *(2006.01)* ***C07C 67/00*** *(2006.01)*

(21) Anmeldenummer: **13177205.5**

(22) Anmeldetag: **19.07.2013**

(54) **Aliphatische langkettige Polykondensate**

Aliphatic long-chain polycondensates

Polycondensats aliphatiques à chaîne longue

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.08.2012 EP 12180147**

(43) Veröffentlichungstag der Anmeldung:
**12.02.2014 Patentblatt 2014/07**

(73) Patentinhaber:
- **BASF SE**
  **67056 Ludwigshafen am Rhein (DE)**
  Benannte Vertragsstaaten:
  **AL AT BE BG CH CZ DE DK EE ES FI FR GB GR HR HU IE IS IT LI LT LU LV MC MK MT NL NO PL PT RO RS SE SI SK SM TR**
- **BASF Schweiz AG**
  **4057 Basel (CH)**
  Benannte Vertragsstaaten:
  **CY**

(72) Erfinder:
- **Mecking, Stefan**
  **78464 Konstanz (DE)**
- **Stempfle, Florian**
  **78467 Konstanz (DE)**

(56) Entgegenhaltungen:
**WO-A1-2011/089256 US-A1- 2006 205 892**

- **DATABASE CA [Online] CHEMICAL ABSTRACTS SERVICE, COLUMBUS, OHIO, US; TANAKA, HIROYUKI: "Electrographic toners containing long-chain polyesters, developers, toner and process cartridges, and electrophotographic apparatuses therefor", XP002713929, gefunden im STN Database accession no. 153:518041 -& JP 2010 231068 A (FUJI XEROX CO., LTD., JAPAN) 14. Oktober 2010 (2010-10-14)**
- **CHO I ET AL: "POLY(TRIACONTAMETHYLENE TRIACONTANEDIOATE) AS POLYETHYLENE ANALOGUE: PROPERTIES AND ENZYMATIC DEGRADATION", MACROMOLECULAR CHEMISTRY AND PHYSICS, WILEY-VCH VERLAG, WEINHEIM, DE, Bd. 198, Nr. 3, 1. März 1997 (1997-03-01), Seiten 861-869, XP000683673, ISSN: 1022-1352, DOI: 10.1002/MACP.1997.021980316**
- **MORITZ EHRENSTEIN ET AL: "Polyamides X.34: A New Class of Polyamides with Long Alkane Segments", MACROMOLECULAR CHEMISTRY AND PHYSICS, Bd. 204, Nr. 13, 1. August 2003 (2003-08-01), Seiten 1599-1606, XP055081831, ISSN: 1022-1352, DOI: 10.1002/macp.200350026**
- **MCKIERNAN R L ET AL: "Synthesis and characterization of polyethylene-like polyurethanes derived from long-chain, aliphatic alpha,omega-diols", POLYMER, ELSEVIER SCIENCE PUBLISHERS B.V, GB, Bd. 43, Nr. 10, 1. Mai 2002 (2002-05-01), Seiten 3007-3017, XP004343304, ISSN: 0032-3861, DOI: 10.1016/S0032-3861(02)00096-4**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- HELEN L NGO ET AL: "Metathesis of Unsaturated fatty Acids: Synthesis of Long-Chain Unsaturated-alpha,omega-Dicarboxylic Acids", JOURNAL OF THE AMERICAN OIL CHEMISTS' SOCIETY, SPRINGER, DE, Bd. 83, Nr. 7, 1. Juli 2006 (2006-07-01), Seiten 629-634, XP002545489, ISSN: 0003-021X, DOI: 10.1007/S11746-006-1249-0

## Beschreibung

Die Erfindung betrifft Polykondensate mit längeren linearen Methylensequenzen in der Hauptkette, Verfahren zu deren Herstellung, deren Verwendung und ein Verfahren zur Herstellung der Monomere.

Nachwachsende Rohstoffe sind als Alternative zu endlichen fossilen Rohstoffen von Interesse. Zudem können damit neuartige Materialien mit vorteilhaften Eigenschaften zugänglich gemacht werden. Hierfür ist es wünschenswert, dass sich die molekulare Struktur des Rohstoffs auch in der Struktur der resultierenden Produkte wiederspiegelt.

Ein attraktives Charakteristikum von Pflanzenölen und Fetten ist die lineare Kohlenwasserstoff-Struktur der (in Form der Triglyceride) enthaltenen Fettsäuren. Diese wird seit langem für die Erzeugung des technischen Kunststoffs Polyamid-11 ausgehend von Rizinolsäure genutzt. In diesem Fall wird allerdings nur ein Teil des Fettsäuremoleküls eingebaut. Die thermische Umlagerung von Rizinolsäure führt unter Spaltung der Fettsäurekette zu Undec-10-ensäure, welche weiter zu Polyamid-11 umgesetzt wird, sowie zur Bildung von Heptanal als Nebenprodukt. Zudem erfordert die zugrundeliegende Umlagerungsreaktion die Anwesenheit der Hydroxygruppe in Nachbarschaft zur Doppelbindung und ist daher unter den derzeit technisch verfügbaren Fettsäuren auf Rizinolsäure und deren Derivate beschränkt. Aus Rizinolsäure ist auch Sebacinsäure zugänglich, welche zu Polykondensaten wie Polyamid-6,10 umgesetzt wird. In diesem Fall entsteht als Nebenprodukt 2-Octanol.

Der Einbau von langkettigen $C_{>20}$-Fettsäuren in Polymere ist erstrebenswert, da beispielsweise lange lineare Methylensequenzen zu wünschenswerten Eigenschaften führen können, wie Kristallinität und damit verbundenen Schmelz- und Kristallisationspunkten, mechanischen und sonstigen Anwendungseigenschaften, Mischungsverhalten in Polymerblends oder einer geringen Wasseraufnahme. Durch Dimerisierung können Fettsäuren zu den sogenannten Dimerfettsäuren umgesetzt werden, welche aber eine verzweigte, nicht-lineare Struktur aufweisen und zu nicht-kristallinen Segmenten führen (Angew. Chem. 2000, 112, 2292). Ein vollständiger linearer Einbau von Fettsäuren in Monomere und daraus erzeugte Polymere ist schwierig zu realisieren, da sich die reaktive Doppelbindung ungesättigter Fettsäuren in der Mitte des Moleküls befindet und die terminale Methylgruppe wenig reaktiv ist.

Bei der Umsetzung von Rohstoffen aus nachwachsenden Quellen ist generell zu bedenken, dass diese meist Verunreinigungen enthalten, welche Umsetzungsverfahren stören können und zu Produktgemischen anstelle von Reinsubstanzen führen. Unterschiedliche Fettsäuren oder deren Derivate weisen, zum Beispiel je nach biologischer Herkunft, häufig ein unterschiedliches Spektrum von Verunreinigungen auf.

WO 2011/089256 offenbart ein Verfahren zur Herstellung von gesättigten oder ungesättigten $\alpha,\omega$-$C_{\geq 23}$-Dicarbonsäuren oder deren Ester mit jeweils einer ungeraden Anzahl an Kohlenstoffatomen im Säureteil durch Hydroxy- oder Alkoxycarbonylierung von einfach oder mehrfach ungesättigten $C_{\geq 22}$-Fettsäureestern mit einer geraden Anzahl an Kohlenstoffatomen im Säureteil sowie Polykondensate auf Basis derartiger Monomere.

In Macromol. Chem. Phys. 2001, 202, 1114 werden u.a. Polykondensate auf Basis des ungesättigten $C_{26}$-Diesters Dimethyl-1,26-hexacos-13-endioat und einem Diol wie Ethylenglykol oder 1,4-Butandiol beschrieben, die Molekulargewichte von 33700 g/mol bzw. 23200 g/mol und Schmelzpunkte von 92 °C bzw. 72 °C aufweisen. Enzymatisch hergestellte Polykondensate auf Basis von Dimethyl-1,26-hexacos-13-endioat und 1,3-Propandiol oder 1,4-Butandiol, mit Molekulargewichten von 2000 g/mol bzw. 7800 g/mol und Schmelzpunkten von 72 °C bzw. 63 °C, werden in Journal of Polymer Science: Part A: Polymer Chemistry 2001, Vol. 39, 1601 offenbart.

In der Literatur ist die Synthese von ungesättigten a,$\omega$-funktionalisierten $C_{26}$-Verbindungen ausgehend von Erucasäure und deren Derivaten über Selbstmetathese sowohl unter Verwendung von homogenen als auch heterogenen Katalysatoren beschrieben (van Dam, P. B.; Mittelmeijer, M. C.; Boelhouwer, C. J. Chem. Soc., Chem. Commun. 1972, 22, 1221-1222; van Dam, P.; Mittelmeijer, M.; Boelhouwer, C. J. Am. Oil Chem. Soc.1974, 51, 389-392; van Dam, P. B.; Mittelmeijer, M. C.; Boelhouwer, C. Fette, Seifen, Anstrichmittel 1974, 76, 264-266; Buchowicz, W.; Mol, J. C. J. Mol. Catal. A: Chem. 1999, 148, 97-103; Warwel, S.; Demes, C.; Steinke, G. J. Polym. Sci., Part A: Polym. Chem. 2001, 39, 1601-1609; Zawadzki, J.; Skupinski React. Kinet. Catal. Lett., 2001, 74 (1), 51-56.). Bei all diesen Reaktionen handelt es sich jedoch um Gleichgewichtsreaktionen, so dass maximal Umsätze von 50 % erreicht werden, wenn nicht eines der Produkte selektiv aus dem Gleichgewicht entfernt werden kann. Durch die von Foglia et al. entwickelte lösungsmittelfreie Selbstmetathese von Erucasäure können diese Einschränkung umgangen werden, da unter den beschriebenen Reaktionsbedingungen die $\alpha,\omega$-$C_{26}$-Disäure ausfällt und so Umsätze von bis zu 70 % erzielt werden können (Ngo, H.; Jones, K.; Foglia, T. J. Am. Oil Chem. Soc. 2006, 83, 629-634; Ngo, H., Foglia, T. A. (United States Department of Agriculture) Patent No.: US 7,534,917 (B1), 2009.).

Darüber hinaus wurde von Warwel et al. ein zweistufiges Verfahren entwickelt, dass ausgehend von Methylerucat durch eine Sequenz von Kreuzmetathese mit Ethylen und anschließender Selbstmetathese der terminal ungesättigten Verbindung ebenfalls zu Dimethyl-1,26-hexacos-13-endioat führt (Warwel, S.; Jägers, H. G.; Thomas, S. Lipid/Fett 1992, 94, 323-328; Warwel, S.; Brüse, F.; Demes, C.; Kunz, M.; Klaas, M. R. g. Chemosphere 2001, 43, 39-48.).

Ansonsten stellt die Synthese von $\alpha,\omega$-funktionalisierten $C_{\geq 26}$-Verbindungen in der organischen Chemie eine große Herausforderung dar. Chemische Syntheserouten solch langkettiger Verbindungen erzeugen oft zahlreiche Nebenprodukte, deren Abtrennung zeit- und kostenintensive Aufreinigungsprozesse erfordert. Dennoch gibt es in der

Literatur einige Beispiele, die sich ausgeklügelten Schutzgruppen-Strategien bedienen und so ausgehend von kleineren Bausteinen über mehrere Stufen zu verschiedenen langkettigen Verbindungen führen. Die Synthese von Dimethyl-1,26-hexacosandioat kann beispielsweise über mehrere Stufen ausgehend von Undecensäuremethylester erfolgen (Günthard, H. H.; Heinemann, S. D.; Prelog, V. Helv. Chim. Acta 1953, 36, 1147-1159.). Daneben kann Dimethyl-1,26-hexacosandioat auch in Anlehnung an das von Hünig et al. beschriebene Verfahren zur Synthese der 1,22-Disäure erhalten werden (Hünig, S.; Lücke, E.; Brenninger, W. Org. Synt. 1973, 5, 533; 1963, 43, 34; Ashikaga, K.; Ito, S.; Yamamoto, M.; Nishijima, Y. J. Am. Chem. Soc. 1988, 110, 198-204.).

Für die Synthese von 1,26-Hexacosandiol finden sich in der Literatur ebenfalls verschiedene mehrstufige Verfahren (Drescher, S.; Meister, A.; Blume, A.; Karlsson, G.; Almgren, M.; Dobner, B., Chem. Eur. J. 2007, 13 (18), 5300-5307.).

Es wurde gefunden, dass gesättigte $\alpha,\omega$-$C_{\geq 26}$-Diester mit einer geraden Anzahl an Kohlenstoffatomen im Säureteil, insbesondere Dimethyl-1,26-hexacosandioat, ausgehend von ungesättigten Säuren, z.B. der gut verfügbaren Fettsäure Erucasäure (cis-13-Docosensäure), durch eine Sequenz aus Selbstmetathese, Veresterung und Reduktion der im Säureteil vorhandenen Doppelbindung(en) hergestellt werden können.

Weiterhin wurde gefunden, dass sich derartige, langkettige Diester, die entsprechenden Carbonsäuren und weitere reaktive Derivate davon, die entsprechenden Alkohole und Amine sowie die davon abgeleiteten Isocyanate als Monomere zur Herstellung von Polykondensaten eignen.

Gegenstand der Erfindung ist daher ein Polykondensat, enthaltend eine oder mehrere Struktureinheiten der Formel (I),

$$-Z-(CH_2)_{2n}-Z'- \qquad (I)$$

worin die Symbole und Indizes folgende Bedeutungen haben:

Z, Z' ist gleich oder verschieden -X-C(=O)~, -C(=O)-HN-$CH_2$~, -C(=O)-O-$CH_2$~,-NH-C(=O)-O-$CH_2$~, -O-C(=O)-NH-$CH_2$~;
X ist O oder NH;
~ gibt die Bindung an die Gruppe $(CH_2)_{2n}$ an; und
n ist = 12.

Weiterhin Gegenstand der Erfindung ist ein Verfahren zur Herstellung des erfindungsgemäßen Polykondensats, wobei mindestens eine monomere Verbindung der Formel (II),

$$Z^1-(CH_2)_{2n}-Z^{1'} \qquad \textbf{(II)}$$

worin

$Z^1$, $Z^{1'}$ ~C(=O)-Q oder ~$CH_2$-NCO ist;
Q gleich oder verschieden OH, Halogen oder $C_1$-$C_{10}$-Alkoxy ist;
~ die Bindung an die Gruppe $(CH_2)_{2n}$ angibt; und
n = 12 ist,

mit mindestens einem Di- oder Polyol oder mindestens einem Di- oder Polyamin polykondensiert wird,
und/oder
mindestens eine monomere Verbindung der Formel (III),

$$Z^2-(CH_2)_{2n}-Z^{2'} \qquad (III)$$

worin

$Z^2$, $Z^{2'}$ gleich oder verschieden HO-$CH_2$~ oder $H_2N$-$CH_2$~ ist;
~ die Bindung an die Gruppe $(CH_2)_{2n}$ angibt; und
n = 12 ist,

mit mindestens einer Di- oder Polycarbonsäure, einem reaktiven Derivat einer solchen Di- oder Polycarbonsäure oder mindestens einem Di- oder Polyisocyanat polykondensiert wird.

Gegenstand der Erfindung ist auch die Verwendung des erfindungsgemäßen Polykondensats in Formteilen, Beschichtungen, Schäumen, Filmen, Folien und/oder Fasern.

Weiterhin Gegenstand der Erfindung sind Formkörper, Beschichtungen, Folien, Schäume und/oder Fasern, enthaltend ein oder mehrere erfindungsgemäße Polykondensate.

Gegenstand der Erfindung ist auch ein Verfahren zur Herstellung eines Dicarbonsäureesters der Formel (IIa),

$$RO\text{-}C(=O)\text{-}(CH_2)_{2n}\text{-}C(=O)\text{-}OR \qquad \text{(IIa)}$$

worin

R gleich oder verschieden $C_1$-$C_{10}$-Alkyl ist; und
n eine ganze Zahl ≥ 12 ist,

umfassend die folgenden Schritte:

i) Umsetzung einer Carbonsäure der Formel (IV),

$$HO\text{-}C(=O)\text{-}(CH_2)_{n-1}\text{-}CH{=}CH\text{-}R^1 \qquad \text{(IV)}$$

worin

$R^1$ $C_1$-$C_{16}$-Alkyl, $C_2$-$C_{16}$-Alkenyl, $C_4$-$C_{16}$-Alkadienyl oder Alkapolyenyl ist; und
n die gleiche Bedeutung wie in der Formel (IIa) hat,

in Anwesenheit eines Ruthenium-Carben-Komplexes, welcher eine Verbindung der Formel (IXa) oder [1,3-Bis(2,4,6-trimethylphenyl)-2-imidazolidinylidene]dichloro-(3-phenyl-1 H-inden-1-ylidene)(pyridyl)ruthenium(II) ist, unter Bildung einer Dicarbonsäure der Formel (V),

$$HO\text{-}C(=O)\text{-}(CH_2)_{n-1}\text{-}CH{=}CH\text{-}(CH_2)_{n-1}\text{-}C(=O)\text{-}OH \qquad \text{(V)}$$

worin
n die gleiche Bedeutung wie in der Formel (IIa) hat;

ii) Umsetzung der Dicarbonsäure der Formel (V)
mit einem oder mehreren Alkoholen der Formel (VI),

$$ROH \qquad \text{(VI)}$$

worin
R die gleiche(n) Bedeutung(en) wie in der Formel (IIa) hat, in Anwesenheit einer Säure
unter Bildung eines Dicarbonsäureesters der Formel (VII),

$$RO\text{-}C(=O)\text{-}(CH_2)_{n-1}\text{-}CH{=}CH\text{-}(CH_2)_{n-1}\text{-}C(=O)\text{-}OR \qquad \text{(VII)}$$

worin
R und n die gleichen Bedeutungen wie in der Formel (IIa) haben;

iii) Umsetzung des Dicarbonsäureesters der Formel (VII)
mit einem Reduktionsmittel
unter Bildung des Dicarbonsäureesters der Formel (IIa).

Weiterhin Gegenstand der Erfindung ist ein Verfahren zur Herstellung eines Diols der Formel (IIIa),

$$HO\text{-}CH_2\text{-}(CH_2)_{2n}\text{-}CH_2\text{-}OH \qquad \text{(IIIa)}$$

worin
n eine ganze Zahl ≥ 12 ist,
umfassend die folgenden Schritte:

i), ii), iii) Herstellung eines Dicarbonsäureesters der Formel (IIa) gemäß des erfindungsgemäßen Verfahrens,

$$RO-C(=O)-(CH_2)_{2n}-C(=O)-OR \qquad (IIa)$$

worin
n die gleiche Bedeutung wie in der Formel (IIIa) hat;

iv) Umsetzung des Dicarbonsäureesters der Formel (IIa)
mit einem Reduktionsmittel
unter Bildung des Diols der Formel (IIIa).

Die Figuren zeigen:

Figur 1: DSC-Analyse von Poly[1,26-hexacosandiyl-1,26-hexacosandioat]

Figur 2: Hochtemperatur-GPC-Analyse von Poly[1,26-hexacosandiyl-1,26-hexacosandioat]

Figur 3: $^1$H-NMR-Spektrum von Poly[1,26-hexacosandiyl-1,26-hexacosandioat]

Erfindungsgemäß ist n eine ganze Zahl $\geq 12$.
Erfindungsgemäß umfasst der Begriff Alkyl lineares und verzweigtes Alkyl.
Erfindungsgemäß versteht man unter dem Begriff Alkoxy eine Alkyloxygruppe, die sich von linearem oder verzweigtem Alkyl ableitet.
Bevorzugte Monomere zur Herstellung der erfindungsgemäßen Polykondensate sind Verbindungen der Formel (II).
Weiterhin bevorzugte Monomere zur Herstellung der erfindungsgemäßen Polykondensate sind Verbindungen der Formel (III).
Vorzugsweise wird das erfindungsgemäße Polykondensat nach dem erfindungsgemäßen Verfahren hergestellt.
In einer bevorzugten Ausführungsform der Erfindung handelt es sich bei der monomeren Verbindung der Formel (II) um einen Dicarbonsäureester der Formel (IIa).
In einer besonders bevorzugten Ausführungsform der Erfindung handelt es sich bei der monomeren Verbindung der Formel (II) um einen Dicarbonsäureester der Formel (IIa), und der Dicarbonsäureester der Formel (IIa) wird nach dem erfindungsgemäßen Verfahren hergestellt.
In einer weiteren bevorzugten Ausführungsform handelt es sich bei der monomeren Verbindung der Formel (III) um ein Diol der Formel (IIIa).
In einer weiteren besonders bevorzugten Ausführungsform handelt es sich bei der monomeren Verbindung der Formel (III) um ein Diol der Formel (IIIa), und das Diol der Formel (IIIa) wird nach dem erfindungsgemäßen Verfahren hergestellt.
In einer besonderen Ausführungsform wird mindestens ein Dicarbonsäureester der Formel (IIa) mit mindestens einem Diol der Formel (IIIa) polykondensiert.
In einer ganz besonderen Ausführungsform wird 1,26-Hexacosandicarbonsäure oder deren Ester, vorzugsweise Dimethyl-1,26-hexacosandioat, mit 1,26-Hexacosandiol polykondensiert.
Als Monomere zur Herstellung der erfindungsgemäßen Polykondensate eignen sich zum Beispiel die entsprechenden $\alpha,\omega$-Dicarbonsäuren und deren Ester. Weiterhin geeignet sind reaktive Derivate dieser Säuren wie Säurehalogenide, aktivierte Ester, Anhydride oder Imidazolide.
Ebenfalls geeignet, beispielsweise in Kombination mit den Disäuren beziehungsweise Diestern, sind die entsprechenden $\alpha,\omega$-Diole, welche aus den Disäuren/Diestern nach bekannten, dem Fachmann geläufigen Reduktionsverfahren erhalten werden können.
Unter Anwendung bekannter Methoden zur Reduktion von Carbonsäurederivaten zu Alkoholen konnte zum Beispiel Dimethyl-1,26-hexacosandioat zu 1,26-Hexacosandiol in guter Ausbeute umgesetzt werden. Beispiele für geeignete Methoden zur Reduktion von Carbonsäuren oder deren Derivaten zu Alkoholen sind die katalytische Hydrierung mit Wasserstoff, die Reduktion durch anorganische hydridische Reagenzien, und die Reduktion durch organische Verbindungen unter Oxidation derselben.
Weiterhin lassen sich die erfindungsgemäß eingesetzten $\alpha,\omega$-Dicarbonsäuren und deren Ester, wie auch die daraus erhältlichen $\alpha,\omega$-Diole, nach bekannten, dem Fachmann geläufigen Methoden in die entsprechenden $\alpha,\omega$-Diamine umwandeln, die wiederum als Monomere bei der Synthese erfindungsgemäßer Polykondensate Anwendung finden können. Beispielhaft genannte bekannte Verfahren zur Umsetzung von Carbonsäuren oder deren Ester zu Aminen sind Amidierung, gefolgt von Wasserabspaltung und Hydrierung; zur Umsetzung von Alkoholen zu Aminen Halogenierung

oder Tosylierung, gefolgt von Umsetzung mit Ammoniak oder alternativ Umsetzung zum Azid gefolgt von Reduktion. Zur Erzeugung von Diisocyanaten können die Amine mit Phosgen oder Phosgenäquivalenten umgesetzt werden.

Ester linearer $C_{26}$ aliphatischer $\alpha,\omega$-Dicarbonsäuren und lineare $C_{26}$ aliphatische $\alpha,\omega$-Diole können zu Polymeren umgesetzt werden, welche ein Gegenstand der Erfindung sind. Die Diester können dabei als solche oder in aktivierter Form, beispielsweise als Säuren, Chloride oder Anhydride, eingesetzt werden. Bevorzugt werden für die Umsetzung zu Polymeren 1,26-Hexacosandicarbonsäure oder deren Ester, 1,26-Hexacosandiol, 1,26-Hexacosandiamin und 1,26-Hexacosandiisocyanat eingesetzt. Besonders bevorzugt werden 1,26-Hexacosandicarbonsäure oder deren Ester eingesetzt.

Neben mindestens einer der im vorangehenden Absatz aufgeführten $\alpha,\omega$-difunktionellen Verbindungen können bei der Herstellung der erfindungsgemäßen Polymere auch weitere mehrfach funktionelle Alkohole, Amine, Säuren oder deren Ester, Chloride oder Anhydride, cyclische Lactame oder Lactone sowie Isocyanate eingesetzt werden. Diese können neben den genannten funktionellen Gruppen auch weitere Heteroatome enthalten. Beispiele für geeignete Alkohole sind Ethylenglykol, 1,3-Propandiol, 1,2-Propandiol, 1,4-Butandiol, Cyclohexandiol, 1,6-Hexandiol, 1,10-Decandiol, 1,12-Dodecandiol, 1,18-Octadecandiol, 1,19-Nonadecandiol, 2,2-Di(4-Hydroxyphenyl)propan, Diethylenglykol, Dihydroxy-terminiertes Polyethylenglykol, Dihydroxy-terminiertes Polytetrahydrofuran und Trimethylolpropan sowie aromatische Di- und Polyhydroxyverbindungen, wie 4,4'-Dihydroxybiphenyl und Bisphenole wie Bisphenol A und Bisphenol AF. Beispiele für geeignete Amine sind Ethylendiamin, Diethylentriamin, 1,2-Diaminopropan, 1,3-Diaminopropan, 1,4-Diaminobutan, 1,5-Diaminopentan, 1,6-Diaminohexan, 1,10-Diaminodecan, 1,12-Diaminododecan sowie aromatische Diamine wie p-Phenylendiamin. Beispiele für geeignete Carbonsäuren sind Maleinsäure, Bernsteinsäure, Fumarsäure, Adipinsäure, Sebacinsäure, 1,19-Nonadecandisäure, Phthalsäure, Terephthalsäure. Beispiele für geeignete Lactame sind $\varepsilon$-Caprolactam, Lauryllactam. Beispiele für geeignete Lactone sind $\varepsilon$-Caprolacton und Butyrolacton. Beispiele für geeignete Diisocyanate sind Hexamethylendiisocyanat und 4,4'-Diphenylmethandiisocyanat.

Bevorzugt wird 1,26-Hexacosandicarbonsäure oder deren Ester von monofunktionellen Alkoholen, insbesondere Dimethyl-1,26-hexacosandioat, in Gegenwart oder Abwesenheit weiterer der im vorangehenden Absatz beispielhaft genannten Dicarbonsäuren oder deren Ester umgesetzt mit einem oder mehreren Diolen, Diaminen, optional in Gegenwart von Lactamen. Geeignete Diole, Diamine und Lactame sind im vorangehenden Absatz beispielhaft genannt.

Besonders bevorzugt wird 1,26-Hexacosandicarbonsäure oder deren Ester von monofunktionellen Alkoholen, insbesondere Dimethyl-1,26-hexacosandioat, mit einer oder mehreren der folgenden Verbindungen polykondensiert: Ethylenglykol, 1,3-Propandiol, 1,4-Butandiol, Cyclohexandiol, 1,6-Hexandiol, 1,10-Decandiol, 1,12-Dodecandiol, 1,18-Octadecandiol, 1,19-Nonadecandiol, 1,23-Tricosandiol, 1,26-Hexacosandiol, 2,2-Di(4-hydroxyphenyl)propan, Diethylenglykol, Dihydroxy-terminiertes Polyethylenglykol, Ethylendiamin, Diethylentriamin, 1,3-Diaminopropan, 1,4-Diaminobutan, 1,5-Diaminopentan, 1,6-Diaminohexan, 1,10-Diaminodecan, 1,12-Diaminododecan, 1,23-Tricosandiamin, 1,26-Hexacosandiamin.

Ebenfalls besonders bevorzugt wird das Monomer der Formel (II), vorzugsweise 1,26-Hexacosandicarbonsäure oder deren Ester von monofunktionellen Alkoholen, insbesondere Dimethyl-1,26-hexacosandioat, zusammen mit Bernsteinsäure, Adipinsäure und/oder Terephthalsäure oder deren Ester (Bernsteinsäureester, Adipinsäureester und/oder Terephthalsäureester) mit einer oder mehreren der folgenden Verbindungen polykondensiert: Ethylenglykol, 1,3-Propandiol, 1,4-Butandiol, Cyclohexandiol, 1,6-Hexandiol, 1,10-Decandiol, 1,12-Dodecandiol, 1,18-Octadecandiol, 1,19-Nonadecandiol, 1,23-Tricosandiol, 1,26-Hexacosandiol, 2,2-Di(4-hydroxyphenyl)propan, Diethylenglykol, Dihydroxy-terminiertes Polyethylenglykol, Ethylendiamin, Diethylentriamin, 1,3-Diaminopropan, 1,4-Diaminobutan, 1,5-Diaminopentan, 1,6-Diaminohexan, 1,10-Diaminodecan, 1,12-Diaminododecan, 1,23-Tricosandiamin, 1,26-Hexacosandiamin.

Polymerisationsverfahren sind dem Fachmann hinlänglich bekannt. Die Polymerisation kann in Anwesenheit oder Abwesenheit von Katalysatoren, Lösungsmitteln, Suspensionsmitteln oder anderen Zusatzstoffen erfolgen. Die Polymerisation kann zum Beispiel in Lösung, Schmelze, Suspension, nicht-wässriger oder wässriger Dispersion erfolgen. Geeignete Katalysatoren sind beispielsweise organische oder anorganische Säuren, sowie Alkoxide, Carboxylate oder Oxide von Übergangs- oder Hauptgruppenmetallen. Die Polymerisation erfolgt bei 0.00001 mbar bis 100 bar Druck. Zur Entfernung flüchtiger Nebenprodukte kann die Polymerisation im Vakuum erfolgen. Das Polymerisationsgemisch kann zwangsgemischt werden, zum Beispiel durch Rührer oder Extruder. Die Polymerisation kann einstufig oder mehrstufig erfolgen.

Optional kann während oder nach der Polymerisation mittels geeigneter Reagenzien Vernetzung erfolgen. Dadurch können bestimmte Eigenschaften, beispielsweise Elastizität oder Fließeigenschaften, erhalten oder verbessert werden.

Weiterhin können die Eigenschaften der erfindungsgemäßen Polykondensate durch Kettenverlängerung, zum Beispiel durch Umsetzung mit Carbonylbiscaprolactam (CBC) und/oder Phenylen-1,4-bis-oxazolin (1,4-PBO), modifiziert werden. Weitere geeignete Methoden der Kettenverlängerung sind beispielsweise die Umsetzung mit mehrfach-funktionellen Isocyanaten. Die erfindungsgemäßen Polymere können als Präpolymere in Polykondensationen und Polyadditionen eingesetzt werden. Zum Beispiel können dihydroxyterminierte erfindungsgemäße Polymere mit mehrfachfunktionellen Isocyanaten oder Carbonsäuren umgesetzt werden.

In einer bevorzugten Ausführungsform entsprechen die Gruppen Z, Z' in der Formel (I) -X-C(=O)- beziehungsweise -X'-C(=O)-, wobei X, X' gleich oder verschieden, vorzugsweise gleich, O oder NH, vorzugsweise O, sind.

Diese Ausführungsform betrifft Polykondensate, enthaltend Struktureinheiten $-X-C(=O)-(CH_2)_{2n}-C(=O)-X'-$ mit n = 12 und X, X' = O oder NH.

Weiterhin bevorzugt sind Polykondensate, in denen Z eine Gruppe $-C(=O)-O-CH_2\sim$, $-C(=O)-NH-CH_2\sim$, $-NH-C(=O)-O-CH_2\sim$ oder $-O-C(=O)-NH-CH_2\sim$ bedeutet, d.h. Polykondensate, enthaltend Struktureinheiten $-M-(CH_2)_{2n+2}-M'-$, wobei n = 12 ist und M, M' eine Amid- (-C(=O)-NH~), Ester- (-C(=O)-O~) oder Urethanfunktion (-NH-C(=O)-O~ oder -O-C(=O)-NH~) bedeuten.

Bevorzugt sind auch Copolyester, die neben einem erfindungsgemäßen Monomer weitere Monomere enthalten, insbesondere Polyester $[\{-A^1-OC(=O)-(CH_2)_{2n}-C(=O)O-\}_x\{-A^1-OC(=O)-A^2-C(=O)O-\}_y]$, wobei n = 12 ist und $A^1$, $A^2$ jeweils einen aliphatischen, cycloaliphatischen, aromatischen oder gemischten Rest mit 2 bis 36 C-Atomen bedeuten. Ein gemischter Rest ist zum Beispiel eine araliphatische Gruppe. x und y bezeichnen jeweils den Molenbruch, d.h. x + y = 1.

Ebenfalls bevorzugt sind auch Copolyamide, die neben einem erfindungsgemäßen Monomer weitere Monomere enthalten, insbesondere Polyamide $[\{-A^3-NHC(=O)-(CH_2)_{2n}-C(=O)NH-\}_x\{-A^3-NHC(=O)-A^4-C(=O)NH-\}_y]$, wobei n = 12 ist und $A^3$, $A^4$ jeweils einen aliphatischen, cycloaliphatischen, aromatischen oder gemischten Rest mit 2 bis 36 C-Atomen bedeuten.

Bevorzugt sind weiterhin erfindungsgemäße Polyester und Polyamide mit y = 0.

Erfindungsgemäß sind Polykondensate mit n = 12.

Besonders bevorzugt sind erfindungsgemäße Polykondensate, bei denen sich zumindest ein Teil der Monomere von 1,26-Hexacosandicarbonsäure ableitet.

Die erfindungsgemäßen Polymere enthalten im Allgemeinen mindestens 1 mol-%, bevorzugt mindestens 10 mol-%, besonders bevorzugt mindestens 50 mol-% mindestens eines Typs der Struktureinheiten der Formel (I), vorzugsweise der Struktureinheiten $-X-C(=O)-(CH_2)_{2n}-C(=O)-X'-$ oder $-M-(CH_2)_{2n+2}-M'-$; mit n = 12, wobei X, X' unabhängig voneinander gleich oder verschieden O oder NH; M, M' unabhängig voneinander gleich oder verschieden eine Amid-, Ester- oder Urethanfunktion bedeuten.

Die erfindungsgemäßen Polykondensate, insbesondere Polyester, weisen im Allgemeinen ein zahlenmittleres Molekulargewicht $M_n$ von 1.000 bis 2.000.000 g/mol, bevorzugt 5.000 bis 200.000 g/mol und besonders bevorzugt 10.000 bis 50.000 g/mol auf.

Ein Vorteil der erfindungsgemäßen Polykondensate, insbesondere Polyester, ist zum einen, dass sie auf der Basis von erneuerbaren Materialien, zum Beispiel Erucasäure oder Erucasäureestern, basierend auf beispielsweise Meerkohl, hergestellt werden können. Als weitere Rohstoffbasis sind darüber hinaus auch Algenöle von Interesse.

Ein weiterer Vorteil der erfindungsgemäßen Polykondensate, insbesondere Polyester, ist, dass sie grundsätzlich als bioabbaubare Polymere geeignet sind. Als bioabbaubare Polymere werden im Sinne der Erfindung solche Materialien bezeichnet, die durch Mikroorganismen, Enzyme oder Hydrolyse, zum Beispiel im Boden, abgebaut werden.

Die Prüfung der biologischen Abbaubarkeit erfolgt nach der DIN-Norm EN 13432. Die bioabbaubaren Materialien müssen innerhalb von 6 bis 10 Wochen in einer Großkompostierung abgebaut werden. In einer weiteren Ausführungsform betrifft die Erfindung somit einen erfindungsgemäßen Polyester, der bioabbaubar ist.

Durch die langkettigen kristallinischen Segmente werden Schmelz- und Kristallisationseigenschaften in wünschenswerter Weise beeinflusst und sind ausreichend hoch für eine thermoplastische Verarbeitung. Erfindungsgemäße Polyester zeigen aufgrund ihrer Kristallinität entsprechend hohe Schmelz- und Kristallinitätspunkte ($T_m$ bzw. $T_c$), die beispielsweise bei $T_m$ > 80 °C und $T_c$ > 70 °C, vorzugsweise $T_m$ > 90 °C und $T_c$ > 75 °C, besonders bevorzugt $T_m$ > 95 °C und $T_c$ > 80 °C, ganz besonders bevorzugt $T_m$ > 100 °C und $T_c$ > 85 °C, insbesondere bevorzugt $T_m$ > 110 °C und $T_c$ > 90 °C liegen können.

Weiterhin zeigen die erfindungsgemäßen Systeme eine geringe Wasseraufnahme.

Die erfindungsgemäßen Polymere lassen sich in zahlreichen Anwendungen vorteilhaft verwenden, beispielsweise in Formteilen, Beschichtungen, Schäumen, Filmen, Folien und Fasern. Die erfindungsgemäßen Polymere können auch in Mischungen mit anderen Kunststoffen verwendet werden.

Als Verfahren zur Verarbeitung der erfindungsgemäßen Polymere seien beispielhaft genannt Spritzguß, Coextrusion, Foliengießen, Blasformen, Folienblasen, Kalandern, Schmelzpressen, Naßspinnen, Trockenspinnen, Schmelzspinnen, Tiefziehen, Pulverbeschichten und Beschichten aus organischer Lösung oder wässriger Dispersion.

Ein weiterer Aspekt der Erfindung betrifft ein Verfahren zur Herstellung eines Dicarbonsäureesters der Formel (IIa),

$$RO-C(=O)-(CH_2)_{2n}-C(=O)-OR \qquad (IIa)$$

worin

R gleich oder verschieden $C_1$-$C_{10}$-Alkyl ist; und

n eine ganze Zahl ≥ 12 ist,

umfassend die oben beschriebenen Schritte i), ii) und iii).

In der Formel (IIa) ist R vorzugsweise gleich.

Bevorzugt ist R in der Formel (IIa) gleich und ausgewählt aus der Gruppe $CH_3$, $C_2H_5$ und $C_4H_9$.

Besonders bevorzugt ist R in der Formel (IIa) $CH_3$.

In der Formel (IIa) ist n vorzugsweise 12.

Gemäß Schritt i) wird eine Carbonsäure der Formel (IV),

$$HO\text{-}C(=O)\text{-}(CH_2)_{n-1}\text{-}CH{=}CH\text{-}R^1 \qquad (IV)$$

worin

$R^1$ $C_1$-$C_{16}$-Alkyl, $C_2$-$C_{16}$-Alkenyl, $C_4$-$C_{16}$-Alkadienyl oder Alkapolyenyl ist; und
n die gleiche Bedeutung wie in der Formel (IIa) hat,

in Anwesenheit eines Ruthenium-Carben-Komplexes umgesetzt, welcher eine Verbindung der Formel (IXa) oder [1,3-Bis(2,4,6-trimethylphenyl)-2-imidazolidinylidene]dichloro-(3-phenyl-1 H-inden-1-ylidene)(pyridyl)ruthenium(II) ist,

Alternativ können auch Carbonsäurederivate wie Ester, insbesondere Mono-, Di-und/oder Triglyceride der entsprechenden Carbonsäure eingesetzt werden.

Vorzugsweise handelt es sich bei Alkapolyenyl um $C_6$-$C_{16}$-Alkatrienyl oder $C_8$-$C_{16}$-Alkatetraenyl, insbesondere $C_6$-$C_{16}$-Alkatrienyl.

Bevorzugt ist $R^1$ in der Formel (IV) $C_1$-$C_{16}$-Alkyl oder $C_4$-$C_{16}$-Alkenyl.

Besonders bevorzugt ist $R^1$ in der Formel (IV) $C_1$-$C_{16}$-Alkyl.

Weiterhin bevorzugt ist $R^1$ in der Formel (IV) n-Octyl, n-Nonyl oder ein entsprechender ein- bis vierfach ungesättigter Rest.

Insbesondere bevorzugt ist $R^1$ in der Formel (IV) n-Octyl.

Bevorzugte Carbonsäuren der Formel (IV) sind Erucasäure und Carbonsäuren der Formel (IV), die aus Algenölen gewonnen werden können.

Eine besonders bevorzugte Carbonsäure der Formel (IV) ist Erucasäure.

Vorzugsweise wird Erucasäure eingesetzt, die (in Form ihrer Ester) aus natürlichen Quellen, beispielsweise aus Pflanzenölen, gewonnen wurde. Insbesondere bevorzugt ist Erucasäure, die (in Form ihrer Ester) aus Samen von erucasäurehaltigen Raps- und Meerkohlsorten, zum Beispiel von Abessinischem Meerkohl, gewonnen wurde.

Als Ruthenium-Carben-Komplexe, kommen beispielsweise solche in Betracht, die sich grundsätzlich als Katalysatoren für die Olefinmetathese eignen (siehe z.B. Grubbs, R.H.; Handbook of Metathesis, Wiley-VCH, Verlag GmbH & Co. KGaA, Weinheim, 2003).

Zum Beispiel können Metall-Carben-Komplexe des Schrock-Typs verwendet werden. Diese sind dem Fachmann bekannt.

Bevorzugte Ruthenium-Carben-Komplexe sind Verbindungen der Formel (VIII), (IX) oder (X),

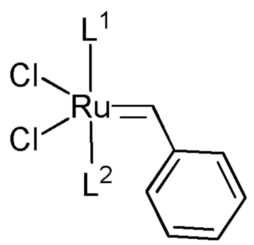

(VIII)

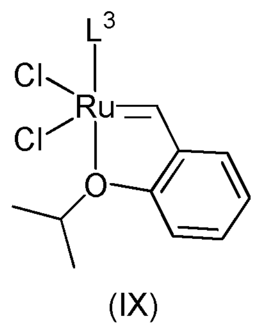

(IX)

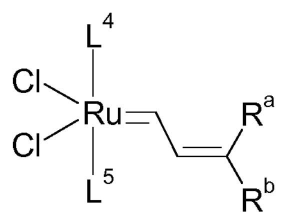

(X)

wobei

$L^1$, $L^2$, $L^3$, $L^4$, $L^5$, jeweils unabhängig voneinander $PPh_3$, $PCy_3$ (Cy = Cyclohexyl),

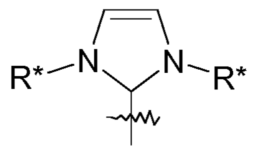

oder

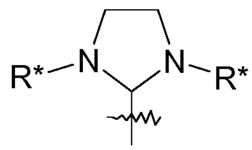

sind;

R* gleich oder verschieden, vorzugsweise gleich 2,4,6-Trimethylphenyl (Mesityl, Mes), 2,6-Diisopropyl, Tolyl oder Adamantyl ist; und

$R^a$, $R^b$ jeweils unabhängig voneinander H, $C_1$-$C_6$-Alkyl oder $C_6$-$C_6$-Aryl sind.

Vorzugsweise sind $R^a$ und $R^b$ Methyl oder Phenyl.

Besonders bevorzugte Ruthenium-Carben-Komplexe sind Verbindungen der Formel (VIII) oder (IX), in denen

$L^1$, $L^2$, $L^3$ jeweils unabhängig voneinander $PCy_3$ oder

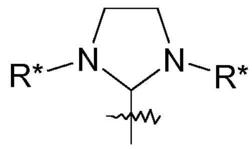

sind; und

R* Mesityl ist.

Ganz besonders bevorzugt sind die Verbindung der Formel (VIIIa) [Grubbs-Katalysator der 2. Generation] und die Verbindung der Formel (IXa) [Grubbs-Hoveyda-Katalysator der 2. Generation], insbesondere die Verbindung der Formel (VIIIa).

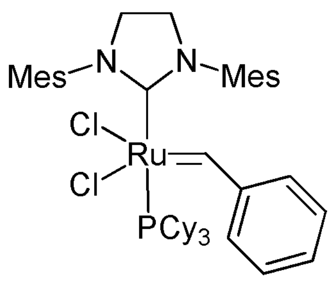

(VIIIa)

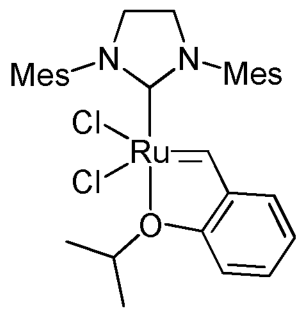

(IXa)

Weiterhin bevorzugte Ruthenium-Carben-Komplexe sind die in WO 2007/010453, WO 00/15339, WO 03/062253 und WO 2008/034552 beschriebenen Ruthenium-Carben-Komplexe. Auf diese Dokumente wird hiermit ausdrücklich Bezug genommen. Sie gelten durch Zitat als Bestandteil der vorliegenden Anmeldung.

Weitere bevorzugte Ruthenium-Carben-Komplexe sind die Verbindungen Dichloro-(3-phenyl-1H-inden-1-ylidene)bis(tricyclohexylphosphine)ruthenium(II), Dichloro-(3-phenyl-1H-inden-1-ylidene)bis(isobutylphobane)ruthenium(II), [1,3-Bis(2,4,6-trimethylphenyl)-2-imidazolidinylidene]dichloro-(3-phenyl-1 H-inden-1-ylidene)(tricyclohexylphosphine)ruthenium(II), [1,3-Bis(2,4,6-trimethylphenyl)-2-imidazolidinylidene]dichloro-(3-phenyl-1H-inden-1-ylidene)(pyridyl)ruthenium(II), [1,3-Bis(2,4,6-trimethylphenyl)-2-imidazolidinylidene]-[2-[[(4-methylphenyl)imino]methyl]-4-nitro-phenolyl]chloro-[3-phenyl-indenylidene]ruthenium(II), [1,3-Bis(2,4,6-trimethylphenyl)-2-imidazolidinylidene]-[2-[[(2-methylphenyl)imino]methyl]-phenolyl]chloro-(3-phenyl-indenylidene)ruthenium(II), [1,3-bis(2,4,6-trimethylphenyl)-2-imidazolidinylidene]dichloro [2-(1-methylacetoxy)phenyl]methyleneruthenium(II). Diese Ruthenium-Carben-Komplexe sind kommerziell erhältlich, z.B. von der Firma Umicore.

Der Ruthenium-Carben-Komplex, wird üblicherweise in Mengen im Bereich von 0,00001 bis 10 mol-%, bevorzugt 0,001 bis 1 mol-%, besonders bevorzugt 0,001 bis 0,5 mol-%, bezogen auf die Menge an verwendeter Carbonsäure der Formel (IV), eingesetzt.

Vorzugsweise wird Schritt i) in Abwesenheit eines Verdünnungsmittels durchgeführt, d.h. die in Schritt i) eingesetzte Carbonsäure der Formel (IV) fungiert als Lösungsmittel. Schritt i) kann aber auch in Anwesenheit eines oder mehrerer Verdünnungsmittel durchgeführt werden. Beispiele für geeignete Verdünnungsmittel sind Ester, Triglyceride, aliphatische und/oder aromatische Kohlenwasserstofffraktionen, beispielsweise n-Butan, iso-Butan, Pentan, Hexan.

Schritt i) wird üblicherweise bei einer Temperatur im Bereich von -50 bis 400 °C, bevorzugt 20 bis 150 °C, besonders bevorzugt 30 bis 100 °C durchgeführt.

Gemäß Schritt ii) wird die in Schritt i) gebildete Dicarbonsäure der Formel (V),

$$HO\text{-}C(=O)\text{-}(CH_2)_{n-1}\text{-}CH{=}CH\text{-}(CH_2)_{n-1}\text{-}C(=O)\text{-}OH \qquad (V)$$

worin
n die gleiche Bedeutung wie in der Formel (IIa) hat,
mit einem oder mehreren Alkoholen der Formel (VI),

$$ROH \qquad (VI)$$

worin
R die gleiche(n) Bedeutung(en) wie in der Formel (IIa) hat,
in Anwesenheit einer Säure umgesetzt.

Vorzugsweise wird ein (1) Alkohol der Formel (VI) eingesetzt.

Bevorzugte Alkohole der Formel (VI) sind Methanol, Ethanol und Butanol.

Ein besonders bevorzugter Alkohol der Formel (VI) ist Methanol.

Bevorzugte Säuren sind Schwefelsäure, Salzsäure, Phosphorsäure, para-Toluolsulfonsäure und feste Säuren. Vorzugsweise handelt es sich bei festen Säuren um auf anorganischen Adsorbentien adsorbierte Säuren.

Besonders bevorzugte Säuren sind Schwefelsäure, Salzsäure und Phosphorsäure.

Ganz besonders bevorzugte Säuren sind Schwefelsäure und Phosphorsäure.

Die Säure wird im Allgemeinen in katalytischen Mengen eingesetzt. Üblicherweise wird die Säure in Mengen im Bereich von 0,00001 bis 50, bevorzugt 0,0001 bis 5, besonders bevorzugt 0,01 bis 2 mol-%, bezogen auf die Menge an verwendeter Dicarbonsäure der Formel (V), eingesetzt.

Vorzugsweise wird Schritt ii) in Abwesenheit eines Verdünnungsmittels durchgeführt, d.h. der in Schritt ii) eingesetzte Alkohol der Formel (VI), z.B. Methanol, fungiert als Lösungsmittel. Schritt ii) kann aber auch in Anwesenheit eines oder mehrerer Verdünnungsmittel durchgeführt werden. Beispiele für geeignete Verdünnungsmittel sind Alkohole, aliphatische und/oder aromatische Kohlenwasserstofffraktionen, beispielsweise n-Butan, iso-Butan, Pentan, Hexan.

Schritt ii) wird üblicherweise bei einer Temperatur im Bereich von -10 bis 300 °C, bevorzugt 20 bis 200 °C, besonders bevorzugt 50 bis 150 °C durchgeführt.

Gemäß Schritt iii) wird der in Schritt ii) gebildete Dicarbonsäureester der Formel (VII),

$$RO\text{-}C(=O)\text{-}(CH_2)_{n-1}\text{-}CH{=}CH\text{-}(CH_2)_{n-1}\text{-}C(=O)\text{-}OR \qquad (VII)$$

worin
R und n die gleichen Bedeutungen wie in der Formel (IIa) haben,
mit einem Reduktionsmittel umgesetzt.

Als Reduktionsmittel für Schritt iii) kommen beispielsweise solche in Betracht, die sich grundsätzlich für die Reduktion von C=C-Doppelbindungen in Gegenwart von Estern eignen (siehe z.B. Sanfilippo, D.; Rylander, P. N. in Ullmann's Encyclopedia of Industrial Chemistry; Wiley-VCH Verlag GmbH & Co. KGaA, 2000).

Ein bevorzugtes Reduktionsmittel für Schritt iii) ist molekularer Wasserstoff in Verbindung mit einem Katalysator, z.B. auf Basis von Palladium, Platin, Nickel, Ruthenium, Eisen, Rhodium oder Cobalt.

Ein besonders bevorzugtes Reduktionsmittel für Schritt iii) ist molekularer Wasserstoff in Verbindung mit einem Katalysator wie Palladium/Aktivkohle (Pd/C) oder mit geträgerten Nickelkatalysatoren.

Ganz besonders bevorzugt ist molekularer Wasserstoff in Verbindung mit Palladium/Aktivkohle (Pd/C) als Katalysator.

Wird molekularer Wasserstoff in Verbindung mit einem Katalysator als Reduktionsmittel eingesetzt, so wird Schritt iii) üblicherweise bei einem Druck im Bereich von 1013 mbar bis 1000 bar, bevorzugt 2 bis 200 bar, besonders bevorzugt 10 bis 100 bar durchgeführt.

Vorzugsweise wird Schritt iii) in Anwesenheit eines Lösungsmittels durchgeführt. Bevorzugte Lösungsmittel

sind Ether wie Tetrahydrofuran, Diethylether, Diisopropylether, Dibutylether, Methyl-tert.-butylether (MTBE), Dioxan, 1,2-Dimethoxyethan (DME); Alkohole wie Methanol, Ethanol; Ester wie Ethylacetat; aliphatische und/oder aromatische Kohlenwasserstofffraktionen, beispielsweise n-Butan, iso-Butan, Pentan, Hexan. Besonders bevorzugt sind THF; Alkohole wie Methanol, Ethanol. Ganz besonders bevorzugt ist THF.

Schritt iii) wird üblicherweise bei einer Temperatur im Bereich von -10 bis 500 °C, bevorzugt 20 bis 200 °C, besonders bevorzugt 50 bis 150 °C durchgeführt.

In einer bevorzugten Ausführungsform ist R $CH_3$; n 12; und $R^1$ n-Octyl.

Ein weiterer Aspekt der Erfindung betrifft ein Verfahren zur Herstellung eines Diols der Formel (IIIa),

$$HO\text{-}CH_2\text{-}(CH_2)_{2n}\text{-}CH_2\text{-}OH \qquad (IIIa)$$

worin
n eine ganze Zahl ≥ 12 ist,
umfassend die oben beschriebenen Schritte i), ii), iii) und iv).

In der Formel (IIIa) ist n vorzugsweise 12.

Gemäß den Schritten i), ii) und iii) wird ein erfindungsgemäßer Dicarbonsäureester der Formel (IIa),

$$RO\text{-}C(=O)\text{-}(CH_2)_{2n}\text{-}C(=O)\text{-}OR \qquad (IIa)$$

worin
n die gleiche Bedeutung wie in der Formel (IIIa) hat,
hergestellt.

Gemäß Schritt iv) wird der Dicarbonsäureester der Formel (IIa) mit einem Reduktionsmittel umgesetzt.

Als Reduktionsmittel für Schritt iv) kommen beispielsweise solche in Betracht, die sich grundsätzlich für die Reduktion von Estern zu Alkoholen eignen (siehe z.B. Angew. Chem. Int. Ed. 2007, 46, 7473).

Bevorzugte Reduktionsmittel für Schritt iv) sind Aluminiumhydride, Borhydride oder molekularer Wasserstoff in Verbindung mit einem Katalysator, z.B. auf Basis von Ruthenium.

Besonders bevorzugte Reduktionsmittel für Schritt iv) sind Lithiumaluminiumhydrid, Diisobutylaluminiumhydrid, Lithiumtriethylborhydrid oder molekularer Wasserstoff in Verbindung mit Dichlorobis[2-(diphenylphosphino)ethylamin]ruthenium als Katalysator.

Ganz besonders bevorzugt sind Lithiumaluminiumhydrid oder molekularer Wasserstoff in Verbindung mit Dichlorobis[2-(diphenylphosphino)ethylamin]ruthenium als Katalysator. Insbesondere bevorzugt ist molekularer Wasserstoff in Verbindung mit Dichloro-bis[2-(diphenylphosphino)ethylamin]ruthenium als Katalysator.

Vorzugsweise wird Schritt iv) in Anwesenheit eines Lösungsmittels durchgeführt. Bevorzugte Lösungsmittel sind Ether wie Tetrahydrofuran, Diethylether, Diisopropylether, Dibutylether, Methyl-tert.-butylether (MTBE), Dioxan, 1,2-Dimethoxyethan (DME); Ester. Insbesondere bevorzugt ist THF.

Schritt iv) wird üblicherweise bei einer Temperatur im Bereich von -10 bis 500 °C, bevorzugt 20 bis 200 °C, besonders bevorzugt 50 bis 150 °C durchgeführt.

In einer Ausführungsform werden die Schritte iii) und iv) zur Herstellung eines Diols der Formel (IIIa) in einem Schritt durchgeführt. Vorzugsweise wird hierfür ein Reduktionsmittel verwendet, das grundsätzlich sowohl für die Reduktion von C=C-Doppelbindungen als auch für die Reduktion von Estern zu Alkoholen geeignet ist.

Die Erfindung wird durch die Beispiele näher erläutert, ohne sie dadurch zu beschränken.

## Beispiele

### 1. Materialien und allgemeine Bemerkungen

Alle Arbeiten wurden in Inertgasatmosphäre unter Verwendung von Argon oder Stickstoff mittels gängiger Standard-Schlenk-Techniken durchgeführt. Apparaturen wurden unter Vakuum ausgeheizt und mit Schutzgas gespült. Tetrahydrofuran wurde über Natrium/Benzophenon unter Inertgasbedingungen getrocknet. Alle anderen Lösungsmittel wurden in technischer Qualität bezogen und ohne weitere Aufreinigung verwendet.

Technische Erucasäure (≈90 %), $[(PCy_3)(n\text{-}C\text{-}C_3H_4N_2Mes_2)Cl_2Ru{=}CHPh]$ (Grubbs Katalysator der 2. Generation), Ethylvinylether, Schwefelsäure, Lithiumaluminiumhydrid und Titan(IV)butoxid (≥ 97 %) wurden von Sigma Aldrich bezogen. Palladium auf Aktivkohle (10 % Pd) stammte von Merck. Alle deuterierten Lösungsmittel wurden von Eurisotop geliefert.

Die NMR-Spektren wurden auf einem Varian Inova 400, einem Bruker Avance 400 sowie einem Bruker Avance DRX 600 Spektrometer aufgenommen. Als interner Standard der $^1H$ bzw. $^{13}C$ NMR-Spektren dienten die Signale der entsprechenden deuterierten Lösungsmittel. Die Hochtemperaturmessungen der Polymerproben wurden in 1,1,2,2-Tetrachlorethan-d2 bei 130 °C durchgeführt.

Die Gaschromatogramme wurden auf einem Perkin Elmer Clarus 500 GC-System mit einer Perkin Elmer Elite-5 Säule (30 m x 0,25 mm x 0,25 μm; 5 % Diphenyl-, 95 % Dimethylpolysiloxan) unter Verwendung eines Flammenionisationsdetektors gemessen. Das Injektions-Volumen des Autosampiers betrug 1 μL. Als Trägergas wurde Helium (99.995 %) verwendet. Die Anfangstemperatur betrug 100 °C und wurde nach einer Minute mit 15 K $min^{-1}$ auf 300 °C erhöht und dort 5 Minuten gehalten (Injektortemperatur 270 °C; Detektortemperatur 280 °C).

Die DSC-Daten wurden auf einem Netzsch DSC 204 F1 im zweiten Aufheizzyklus bei einer Heiz- bzw. Kühlrate von 10 K $min^{-1}$ ermittelt.

Die Hochtemperatur-GPC-Analysen wurden in 1,2,4-Trichlorbenzol bei 160 °C mit einer Flussgeschwindigkeit von 1 mL $min^{-1}$ auf einem Polymer Laboratories 220 Gerät mit einem Brechungsindex-, einem Viskositäts-, und einem Lichtstreudetektor (15° und 90°) auf Olexis-Säulen durchgeführt. Alle angegebenen Daten wurden direkt gegen PE Standards ausgewertet.

## 2. Synthese der Monomere

### Dimethyl-1,26-hexacos-13-endioat

Erucasäure (45.0 g; 133.0 mmol) wurde in einem 250 mL Glasreaktor mit mechanischem Rührwerk vorgelegt und bei 45 °C geschmolzen. Nach sorgfältigem Entgasen wurde $[(PCy_3)(\eta\text{-}C\text{-}C_3H_4N_2Mes_2)Cl_2Ru{=}CHPh]$ (Grubbs Katalysator der 2. Generation) (113 mg; 0.133 mmol) zugegeben. Anschließend wurde die Schmelze 15 Minuten bei 45 °C gerührt, wobei ein beiger Feststoff ausfiel. Danach wurde die Reaktion mit Ethylvinylether (10 mL) gestoppt und nach Zugabe von 100 mL Chloroform für 30 Minuten bei 70 °C gerührt. Um Katalysatorrückstände abzutrennen, wurde die nun klare gelbe Lösung über Kieselgur filtriert. Nach Entfernen des Lösungsmittels am Vakuum und Umkristallisation aus Ethylacetat konnte die Disäure als Rohprodukt erhalten werden.

Zur Veresterung wurde die Disäure in 150 mL Methanol suspendiert und mit 8 Tropfen konzentrierter Schwefelsäure versetzt. Danach wurde das Reaktionsgemisch für eine halbe Stunde bei 65 °C gerührt. Nach mehrmaliger Umkristallisation aus Methanol wurde reines Dimethyl-1,26-hexacos-13-endioat (14,9 g; 49 %) erhalten.

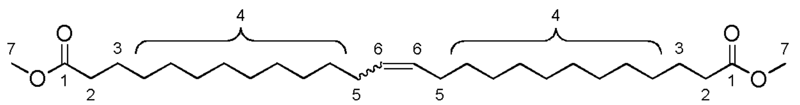

$^1H$-NMR ($CDCl_3$, 25 °C, 400 MHz): δ 5.41-5.35 (m, 2H, H-6), 3.66 (s, 6H, H-7), 2.30 (t, $^3J_{H\text{-}H}$ = 7.4 Hz, 4H, H-2), 2.01-1.91 (m, 4H, H-5), 1.66-1.58 (m, 4H, H-3), 1.37-1.22 (m, 32H, H-4).

$^{13}C$-NMR ($CDCl_3$, 25 °C, 400 MHz): δ 174.47 (C-1), 130.49 (C-6), 51.55 (C-7), 34.26 (C-2), 32.75 (C-5), 30.08-29.00 (C-4), 25.11 (C-3).

### Dimethyl-1,26-hexacosandioat

Dimethyl-1,26-hexacos-13-endioat (14.9 g; 33.0 mmol) wurde in 90 mL THF gelöst und nach Zugabe von 1.48 g Pd/C (10 % Pd) in einen mechanisch gerührten 250 mL Druckreaktor mit Doppelmantel kanüliert. Unter Rühren (1000 rpm) wurden 60 bar Wasserstoff aufgepresst und der Außenmantel des Reaktors mit Hilfe eines Thermostaten erwärmt. Die gewünschte Reaktionstemperatur von 80 °C wurde dabei über einen in das Reaktionsgemisch eintauchenden Thermofühler verfolgt und automatisch reguliert. Nach 66 Stunden wurde der Reaktor auf Raumtemperatur abgekühlt und anschließend vorsichtig entspannt. Um Katalysatorrückstande zu entfernen wurde das Reaktionsgemisch auf ungefähr 50 °C erwärmt und heiß über Kieselgur filtriert. Nach Entfernen des Lösungsmittels am Vakuum und Umkristallisation aus Methanol wurde Dimethyl-1,26-hexacosandioat (12.2 g; 82 %) als weißer Feststoff erhalten.

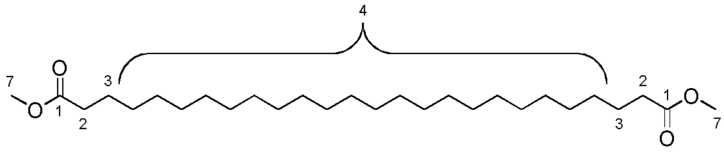

$^1H$-NMR ($CDCl_3$, 25 °C, 400 MHz): δ 3.66 (s, 6H, H-7), 2.30 (t, $^3J_{H\text{-}H}$ = 7.4 Hz, 4H, H-2), 1.66-1.56 (m, 4H, H-3), 1.34-1.20 (m, 40H, H-4).

$^{13}C$-NMR ($CDCl_3$, 25 °C, 101 MHz): δ 174.49 (C-1), 51.57 (C-7), 34.27 (C-2), 30.23-29.02 (C-4), 25.12 (C-3).

**1,26-Hexacosandiol**

Dimethyl-1,26-hexacosandioat (5.9 g; 13.0 mmol) wurde in 100 mL Tetrahydrofuran gelöst und langsam zu einer gekühlten Suspension von $LiAlH_4$ in 40 mL Tetrahydrofuran getropft. Nach beendeter Zugabe wurde das Reaktionsgemisch 12 Stunden unter Rückfluss erhitzt und dann auf Raumtemperatur abgekühlt. Die Reaktion wurde durch Zugabe von 2.0 mL Wasser, 2.0 mL NaOH (15 %) und weiteren 6.0 mL Wasser gestoppt und anschließend bei ungefähr 50 °C heiß über Kieselgur abfiltriert. Nach Entfernen des Lösungsmittels am Vakuum und Umkristallisation aus Chloroform wurden 4.4 g Hexacosan-1,26-diol (85 %) erhalten.

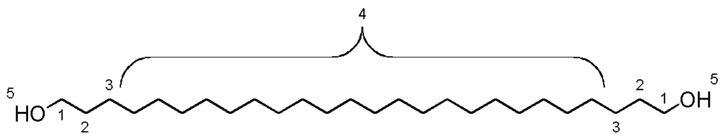

$^1$H-NMR ($CDCl_3$, 50 °C, 400 MHz): 3.64 (t, $^3$JH-H = 6.5 Hz, 4H, H-1), 1.61-1.53 (m, 4H, H-2), 1.41-1.24 (m, 44H, H-3 and H-4), 1.18 (br s, 2H, H-4).
$^{13}$C-NMR ($CDCl_3$, 50 °C, 101 MHz): $\delta$ 63.27 (C-1), 33.06 (C-2), 30.10-29.22 (C-4), 25.95 (C-3).

3. Synthese und Charakterisierung der Polymere

Polymerisation

Die Synthese von Polyester-26.26 erfolgte in einem 100 mL Polymerisationsschlenk mit Seitenhahn und mechanischem Rührwerk. Eine effektive Durchmischung der Polymerschmelze wurde dabei durch ein helikales Rührblatt erreicht. Unter Argonatmosphäre wurden die beiden Monomere (4.0 mmol Dimethyl-1,26-hexacosandioat und 4.0 mmol Hexacosan-1,26-diol) vorgelegt und bei 130 °C geschmolzen. Nach der Zugabe von Titan(IV)butoxid (27 mg, 0.08 mmol) wurde die Temperatur innerhalb von sieben Stunden langsam auf 200 °C erhöht. Anschließend wurde die Reaktionstemperatur auf 180 °C gesenkt und die Polymerschmelze für weitere 19 Stunden im Vakuum (0.01 mbar) gerührt.
Poly[1,26-hexacosandiyl-1,26-hexacosandioat]: $T_m$ 114 °C, $T_c$ 92 °C, $LIH_m$ 160 J $g^{-1}$ Figur 1 zeigt eine DSC-Analyse von Poly[1,26-hexacosandiyl-1,26-hexacosandioat].
Figur 2 zeigt eine Hochtemperatur-GPC-Analyse (1,2,4-Trichlorbenzol, 160 °C) von Poly[1,26-hexacosandiyl-1,26-hexacosandioat].

Mittlere Molekulargewichte:

| | | | |
|---|---|---|---|
| Mp: 55814 | Mn: 24435 | Mv: 55812 | Mw: 62366 |
| Mz: 117553 | Mz+1: 181691 | PD: 2.5523 | |

Figur 3 zeigt ein $^1$H-NMR-Spektrum (600 MHz, $C_2D_2Cl_4$, 130 °C) von Poly[1,26-hexacosandiyl-1,26-hexacosandioat].
Die DSC-Analyse zeigt einen Schmelzpunkt $T_m$ von 114 °C, einen Kristallinitätspunkt $T_c$ von 92 °C und eine Schmelzenthalpie $\Delta H_m$ von 160 J $g^{-1}$.]
Die Hochtemperatur-GPC-Analyse zeigt ein zahlenmittleres Molekulargewicht $M_n$ von üblicherweise 2.4 x $10^4$ g $mol^{-1}$ ($M_w/M_n$ = 2.6); dieses Ergebnis stimmt mit dem über NMR-spektroskopische Analyse der Endgruppen ermittelten Molekulargewicht überein.

**Molekulargewichtsbestimmung mittels $^1$H-NMR Spektroskopie**

Das zahlenmittlere Molekulargewicht sowie der Polymerisationsgrad ($DP_n$) können aus den $^1$H NMR-Spektren über die Signalintensitäten der Endgruppen im Vergleich zu den Intensitäten der Signale in Nachbarschaft der Estergruppen der Struktureinheit der Polymerkette abgeschätzt werden.
Im Allgemeinen ist davon auszugehen, dass drei verschiedene Arten von Endgruppen im Polymer vorhanden sein können (Endgruppen aus den Monomeren sowie dem Katalysator). In diesem Fall jedoch finden sich nur zwei verschiedene Endgruppen:

- E1: Hydroxyendgruppe (3.67 ppm, t, 2H)
- E2: Butylesterendgruppe (0.96 ppm, t, 3H)

Der Polymerisationsgrad kann über die Integrale der Signale dieser Endgruppen und beispielsweise des Integrals E3 der Methylengruppe neben der Carbonylgruppe nach folgender Gleichung berechnet werden:

$$DP_n = \frac{\int E_3}{\left(\frac{1}{2}\int E_1 + \frac{1}{3}\int E_2\right)} \approx 52$$

Figur 1 zeigt eine DSC-Analyse von Poly[1,26-hexacosandiyl-1,26-hexacosandioat].

Figur 2 zeigt eine Hochtemperatur-GPC-Analyse (1,2,4-Trichlorbenzol, 160 °C) von Poly[1,26-hexacosandiyl-1,26-hexacosandioat].

Mittlere Molekulargewichte:

| | | | |
|---|---|---|---|
| Mp: 55814 | Mn: 24435 | Mv: 55812 | Mw: 62366 |
| Mz: 117553 | Mz+1: 181691 | PD: 2.5523 | |

Figur 3 zeigt ein $^1$H-NMR-Spektrum (600 MHz, $C_2D_2Cl_4$, 130 °C) von Poly[1,26-hexacosandiyl-1,26-hexacosandioat].

Die DSC-Analyse zeigt einen Schmelzpunkt $T_m$ von 114 °C, einen Kristallinitätspunkt $T_c$ von 92 °C und eine Schmelzenthalpie $\Delta H_m$ von 160 J $g^{-1}$.]

Die Hochtemperatur-GPC-Analyse zeigt ein zahlenmittleres Molekulargewicht $M_n$ von üblicherweise 2.4 x $10^4$ g $mol^{-1}$ ($M_w/M_n$ = 2.6); dieses Ergebnis stimmt mit dem über NMR-spektroskopische Analyse der Endgruppen ermittelten Molekulargewicht überein.

**Molekulargewichtsbestimmung mittels $^1$H-NMR Spektroskopie**

Das zahlenmittlere Molekulargewicht sowie der Polymerisationsgrad ($DP_n$) können aus den $^1$H NMR-Spektren über die Signalintensitäten der Endgruppen im Vergleich zu den Intensitäten der Signale in Nachbarschaft der Estergruppen der Struktureinheit der Polymerkette abgeschätzt werden.

Im Allgemeinen ist davon auszugehen, dass drei verschiedene Arten von Endgruppen im Polymer vorhanden sein können (Endgruppen aus den Monomeren sowie dem Katalysator). In diesem Fall jedoch finden sich nur zwei verschiedene Endgruppen:

- E1: Hydroxyendgruppe (3.67 ppm, t, 2H)
- E2: Butylesterendgruppe (0.96 ppm, t, 3H)

Der Polymerisationsgrad kann über die Integrale der Signale dieser Endgruppen und beispielsweise des Integrals E3 der Methylengruppe neben der Carbonylgruppe nach folgender Gleichung berechnet werden:

$$DP_n = \frac{\int E_3}{\left(\frac{1}{2}\int E_1 + \frac{1}{3}\int E_2\right)} \approx 52$$

**Patentansprüche**

**1.** Polykondensat, enthaltend eine oder mehrere Struktureinheiten der Formel (I),

$$-Z-(CH_2)_{2n}-Z'- \qquad (I)$$

worin die Symbole und Indizes folgende Bedeutungen haben:

Z, Z' ist gleich oder verschieden -X-C(=O)~ -C(=O)-HN-$CH_2$~, -C(=O)-O-$CH_2$~, -NH-C(=O)-O-$CH_2$~, -O-C(=O)-NH-$CH_2$~;
X ist O oder NH;
~ gibt die Bindung an die Gruppe $(CH_2)_{2n}$ an; und
n = 12.

**2.** Polykondensat gemäß Anspruch 1, enthaltend Struktureinheiten $-X-C(=O)-(CH_2)_{2n}-C(=O)-X'-$ mit n = 12 und X, X' = O oder NH.

**3.** Polykondensat gemäß Anspruch 1, enthaltend Struktureinheiten $-M-(CH_2)_{2n+2}-M'-$ wobei n = 12 ist und M, M' eine Amid- (-C(=O)-NH~), Ester- (-C(=O)-O~) oder Urethanfunktion (-NH-C(=O)-O~ oder -O-C(=O)-NH~) bedeutet.

**4.** Polyester gemäß Anspruch 1 oder 2 mit Struktureinheiten $[\{-A^1-OC(=O)-(CH_2)_{2n}-C(=O)O-\}_x\{-A^1-OC(=O)-A^2-C(=O)O-\}_y]$, wobei n = 12 ist und $A^1$, $A^2$ gleich oder verschieden einen aliphatischen, cycloaliphatischen, aromatischen oder gemischten Rest mit 2 bis 36 C-Atomen bedeuten und x + y = 1 gilt.

**5.** Polyamid gemäß Anspruch 1 oder 2 mit Struktureinheiten $[\{-A^3-NHC(=O)-(CH_2)_{2n}-C(=O)NH-\}_x\{-A^3-NHC(=O)-A^4-C(=O)NH-\}_y]$, wobei n = 12 ist und $A^3$, $A^4$ gleich oder verschieden einen aliphatischen, cycloaliphatischen, aromatischen oder gemischten Rest mit 2 bis 36 C-Atomen bedeuten und x + y = 1 gilt.

**6.** Polykondensat gemäß Anspruch 4 oder 5, wobei y = 0 gilt.

**7.** Polykondensat gemäß einem der Ansprüche 1 bis 6, enthaltend mindestens 1 mol-% an einer oder mehreren Struktureinheiten der Formel (I).

**8.** Verfahren zur Herstellung eines Polykondensats gemäß einem der Ansprüche 1 bis 7, wobei mindestens eine monomere Verbindung der Formel (II),

$$Z^1-(CH_2)_{2n}-Z^{1'} \qquad (II)$$

worin

$Z^1$, $Z^{1'}$ ~C(=O)-Q oder $\sim CH_2-NCO$ ist;
Q gleich oder verschieden OH, Halogen oder $C_1$-$C_{10}$-Alkoxy ist;
~ die Bindung an die Gruppe $(CH_2)_{2n}$ angibt; und
n = 12 ist,

mit mindestens einem Di- oder Polyol oder mindestens einem Di- oder Polyamin polykondensiert wird,
und/oder
mindestens eine monomere Verbindung der Formel (III),

$$Z^2-(CH_2)_{2n}-Z^{2'} \qquad (III)$$

worin

$Z^2$, $Z^{2'}$ gleich oder verschieden $HO-CH_2\sim$ oder $H_2N-CH_2\sim$ ist;
~ die Bindung an die Gruppe $(CH_2)_{2n}$ angibt; und
n = 12 ist,

mit mindestens einer Di- oder Polycarbonsäure, einem reaktiven Derivat einer solchen Di- oder Polycarbonsäure oder mindestens einem Di- oder Polyisocyanat polykondensiert wird.

**9.** Verwendung eines Polykondensats gemäß einem der Ansprüche 1 bis 7 in Formteilen, Beschichtungen, Schäumen, Filmen, Folien und/oder Fasern.

**10.** Formkörper, Beschichtungen, Folien, Schäume und/oder Fasern, enthaltend ein oder mehrere Polykondensate gemäß einem der Ansprüche 1 bis 7.

**11.** Verfahren zur Herstellung eines Dicarbonsäureesters der Formel (IIa),

$$RO-C(=O)-(CH_2)_{2n}-C(=O)-OR \qquad (IIa)$$

worin

R gleich oder verschieden $C_1$-$C_{10}$-Alkyl ist; und
n eine ganze Zahl ≥ 12 ist,

umfassend die folgenden Schritte:

i) Umsetzung einer Carbonsäure der Formel (IV),

$$HO\text{-}C(=O)\text{-}(CH_2)_{n-1}\text{-}CH=CH\text{-}R^1 \qquad (IV)$$

worin
$R^1$ $C_1$-$C_{16}$-Alkyl, $C_2$-$C_{16}$-Alkenyl, $C_4$-$C_{16}$-Alkadienyl oder Alkapolyenyl ist; und
n die gleiche Bedeutung wie in der Formel (IIa) hat,
in Anwesenheit eines Ruthenium-Carben-Komplexes, welcher eine Verbindung der Formel (IXa)

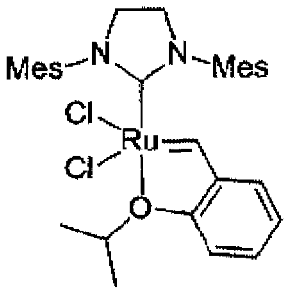

(IXa) ¶

oder [1,3-Bis(2,4,6-trimethylphenyl)-2-imidazolidinylidene]dichloro-(3-phenyl-1H-inden-1-ylidene)(pyridyl)ruthenium(II) ist,
unter Bildung einer Dicarbonsäure der Formel (V),

$$HO\text{-}C(=O)\text{-}(CH_2)_{n-1}\text{-}CH=CH\text{-}(CH2)_{n-1}\text{-}C(=O)\text{-}OH \qquad (V)$$

worin
n die gleiche Bedeutung wie in der Formel (IIa) hat;
ii) Umsetzung der Dicarbonsäure der Formel (V)
mit einem oder mehreren Alkoholen der Formel (VI),

$$ROH \qquad (VI)$$

worin
R die gleiche(n) Bedeutung(en) wie in der Formel (IIa) hat,
in Anwesenheit einer Säure
unter Bildung eines Dicarbonsäureesters der Formel (VII),

$$RO\text{-}C(=O)\text{-}(CH_2)_{n-1}\text{-}CH=CH\text{-}(CH_2)_{n-1}\text{-}C(=O)\text{-}OR \qquad (VII)$$

worin
R und n die gleichen Bedeutungen wie in der Formel (IIa) haben;
iii) Umsetzung des Dicarbonsäureesters der Formel (VII)
mit einem Reduktionsmittel
unter Bildung des Dicarbonsäureesters der Formel (IIa).

**12.** Verfahren zur Herstellung eines Diols der Formel (IIIa),

$$HO\text{-}CH_2\text{-}(CH_2)_{2n}\text{-}CH_2\text{-}OH \qquad (IIIa)$$

worin

n eine ganze Zahl $\geq 12$ ist,
umfassend die folgenden Schritte:

i), ii), iii) Herstellung eines Dicarbonsäureesters der Formel (IIa) gemäß Anspruch 11,

$$RO\text{-}C(=O)\text{-}(CH_2)_{2n}\text{-}C(=O)\text{-}OR \qquad (IIa)$$

worin
n die gleiche Bedeutung wie in der Formel (IIIa) hat;
iv) Umsetzung des Dicarbonsäureesters der Formel (IIa)
mit einem Reduktionsmittel
unter Bildung des Diols der Formel (IIIa).

**13.** Verfahren gemäß Anspruch 11 oder 12, wobei R $CH_3$ ist; n 12 ist; und $R^1$ n-Octyl ist.

## Claims

**1.** A polycondensate comprising one or more structural units of formula (I),

$$\text{-}Z\text{-}(CH_2)_{2n}\text{-}Z'\text{-} \qquad (I)$$

where

Z and Z' are each -X-C(=O)~, -C(=O)-HN-$CH_2$~, - C(=O)-O-$CH_2$~, -NH-C(=O)-O-$CH_2$~, -O-C(=O)-NH-$CH_2$~ and are the same or different;
X is 0 or NH;
~ is the bond to the $(CH_2)_{2n}$ group; and
n = $\geq 12$.

**2.** The polycondensate according to claim 1 comprising structural units -X-C(=O)-$(CH_2)_{2n}$-C(=O)-X'- where n = 12 and X, X' = 0 or NH.

**3.** The polycondensate according to claim 1 comprising structural units -M-$(CH_2)_{2n+2}$-M' - where n = 12 and M and M' are each an amide function - (-C(=O)-NH~), an ester function -(-C(=O)-O~) or a urethane function (-NH-C(=O)-O~ oder -O-C(=O)-NH~).

**4.** A polyester according to claim 1 or 2 with structural units [{-$A^1$-OC(=O)-$(CH_2)_{2n}$-C(=O)O-}$_x${-$A^1$-OC(=O)-$A^2$-C(=O)O-}$_y$], where n = 12 and $A^1$ and $A^2$ are each an aliphatic, cycloaliphatic, aromatic or mixed radical of 2 to 36 carbon atoms and are the same or different, and x + y = 1 applies.

**5.** A polyamide according to claim 1 or 2 with structural units [{-$A^3$-NHC(=O)-$(CH_2)_{2n}$-C(=O)NH-}$_x${-$A^3$-NHC(=O)-$A^4$-C(=O)NH-}$_y$], where n = 12 and $A^3$ and $A^4$ are each an aliphatic, cycloaliphatic, aromatic or mixed radical of 2 to 36 carbon atoms and are the same or different, and x + y = 1 applies.

**6.** The polycondensate according to claim 4 or 5 where in y = 0 applies.

**7.** The polycondensate according to any of claims 1 to 6 comprising at least 1 mol% of one or more structural units of formula (I).

**8.** A process for producing a polycondensate according to any of claims 1 to 7, which process comprises polycondensing at least one monomeric compound of formula (II),

$$Z^1\text{-}(CH_2)_{2n}\text{-}Z^{1'} \qquad (II),$$

where

$Z^1$ and $Z^{1'}$ are each ~C(=O)-Q or ~$CH_2$-NCO;

Q in each occurrence is OH, halogen or $C_1$-$C_{10}$ alkoxy and the same or different;
~ is the bond to the $(CH_2)_{2n}$ group; and
n = 12,

with at least one di- or polyol or at least one di-or polyamine,
and/or
at least one monomeric compound of formula (III)

$$Z^2\text{-}(CH_2)_{2n}\text{-}Z^{2'} \qquad \text{(III)},$$

where

$Z^2$ and $Z^{2'}$ are each $HO\text{-}CH_2$~ or $H_2N\text{-}CH_2$~ and are the same or different;
~ is the bond to the $(CH_2)_{2n}$ group; and
n = 12,

with one or more than one di- or polycarboxylic ac-id, with a reactive derivative thereof or with at least one di- or polyisocyanate.

**9.** A method of using a polycondensate according to any of claims 1 to 7 in moldings, coatings, foams, supported films, unsupported film or sheet, and/or fibers.

**10.** A molded article, a coating, an unsupported film or sheet, a foam or a fiber comprising one or more polycondensates according to any of claims 1 to 7.

**11.** A process for producing a dicarboxylic ester of formula (IIa),

$$RO\text{-}C(=O)\text{-}(CH_2)_{2n}\text{-}C(=O)\text{-}OR \qquad \text{(IIa)}$$

where

R in each occurrence is $C_1$-$C_{10}$ alkyl and the same or different; and
n is an integer ≥ 12,

comprising the steps of:

i) reacting a carboxylic acid of formula (IV),

$$HO\text{-}C\ (=O)\text{-}(CH_2)_{n-1}\text{-}CH{=}CH\text{-}R^1 \qquad \text{(IV)}$$

where

$R^1$ is $C_1$-$C_{16}$ alkyl, $C_2$-$C_{16}$ alkenyl, $C_4$-$C_{16}$ alkadienyl or alkapolyenyl; and
n has the same meaning as in formula (IIa),

in the presence of a ruthenium-carbene complex which is a compound of formula (IXA) [1,3-bis(2,4,6-trimethylphenyl)-2-imidazolidinylidene]dichloro-(3-phenyl-1H-inden-1-ylidene)(pyridyl)ruthenium(II),
to form a dicarboxylic acid of formula (V),

$$HO\text{-}C(=O)\text{-}(CH_2)_{n-1}\text{-}CH{=}CH\text{-}(CH_2)_{n-1}\text{-}C(=O)\text{-}OH \qquad \text{(V)}$$

where
n has the same meaning as in formula (IIa);
ii) reacting the dicarboxylic acid of formula (V) with one or more alcohols of formula (VI),

$$ROH \qquad \text{(VI)}$$

where
R has the same meaning(s) as in formula (IIa), in the presence of an acid to form a dicarboxylic ester of formula (VII),

$$RO-C(=O)-(CH_2)_{n-1}-CH=CH-(CH_2)_{n-1}-C(=O)-OR \quad (VII)$$

where
R and n each have the same meanings as in formula (IIa);
iii) reacting the dicarboxylic ester of formula (VII)

with a reducing agent
to form the dicarboxylic ester of formula (IIa).

**12.** A process for producing a diol of formula (IIIa),

$$HO-CH_2-(CH_2)_{2n}-CH_2-OH \quad (IIIA)$$

where
n is an integer ≥ 12,
comprising the steps of:

i), ii), iii) producing a dicarboxylic ester of formula (IIa) according to claim 11,

$$RO-C(=O)-(CH_2)_{2n}-C(=O)-OR \quad (IIa)$$

where
n has the same meaning as in formula (IIIa);
iv) reacting the dicarboxylic ester of formula (IIa)
with a reducing agent
to form the diol of formula (IIIa).

**13.** The process according to claim 11 or 12 wherein R is $CH_3$; n is 12 and $R^1$ is n-octyl.

## Revendications

**1.** Polycondensat, contenant une ou plusieurs unités structurales de formule (I)

$$-Z-(CH_2)_{2n}-Z'- \quad (I)$$

dans laquelle les symboles et les indices ont les significations suivantes :

Z, Z' sont identiques ou différents, et signifient -X-C(=O)~, -C(=O)-HN-$CH_2$~, -C(=O)-O-$CH_2$~, -NH-C(=O)-O-$CH_2$~, -O-C(=O)-NH-$CH_2$~;
X signifie 0 ou NH;
~ indique la liaison au groupe $(CH_2)_{2n}$; et
n = 12.

**2.** Polycondensat selon la revendication 1, contenant des unités structurales -X-C(=O)-$(CH_2)_{2n}$-C(=O)-X'- avec n = 12 et X, X' = O ou NH.

**3.** Polycondensat selon la revendication 1, contenant des unités structurales -M-$(CH_2)_{2n+2}$-M'-, avec n = 12 et M, M' = une fonction amide (-C(=O)-NH~), ester (-C(=O)-O~) ou uréthane (-NH-C(=O)-O~ ou -O-C(=O)-NH~).

**4.** Polyester selon la revendication 1 ou 2, contenant des unités structurales $[\{-A^1-OC(=O)-(CH)_{2n}-C(=O)O-\}_x\{-A^1-OC(=O)-A^2-C(=O)O-\}_y]$, avec n = 12 et $A^1$, $A^2$ identiques ou différents, signifiant un radical aliphatique, cycloaliphatique, aromatique ou mixte de 2 à 36 atomes C, et x + y = 1.

5. Polyamide selon la revendication 1 ou 2, contenant des unités structurales [{-$A^3$-NHC(=O)-$(CH_2)_{2n}$-C(=O)NH-}$_x${-$A^3$-NHC(=O)-$A^4$-C(=O)NH-}$_y$], avec n = 12 et $A^3$, $A^4$ identiques ou différents, signifiant un radical aliphatique, cycloaliphatique, aromatique ou mixte de 2 à 36 atomes C, et x + y = 1.

6. Polycondensat selon la revendication 4 ou 5, dans lequel y = 0.

7. Polycondensat selon l'une quelconque des revendications 1 à 6, contenant au moins 1 % en moles d'une ou de plusieurs unités structurales de formule (I).

8. Procédé de fabrication d'un polycondensat selon l'une quelconque des revendications 1 à 7, selon lequel au moins un composé monomère de formule (II)

$$Z^1\text{-}(CH_2)_{2n}\text{-}Z^{1'} \qquad \text{(II)}$$

dans laquelle

$Z^1$, $Z^{1'}$ signifient ~C(=O)-Q ou ~$CH_2$-NCO ;
Q identiques ou différents, signifient OH, halogène ou alcoxy en $C_1$-$C_{10}$;
~ indique la liaison au groupe $(CH_2)_{2n}$ ; et
n = 12,

est polycondensé avec au moins un di- ou polyol ou au moins une di- ou polyamine,
et/ou
au moins un composé monomère de formule (III)

$$Z^2\text{-}(CH_2)_{2n}\text{-}Z^{2'} \qquad \text{(III)}$$

dans laquelle

$Z^2$, $Z^{2'}$ identiques ou différents, signifient HO-$CH_2$- ou $H_2N$-$CH_2$~;
~ indique la liaison au groupe $(CH_2)_{2n}$ ; et
n = 12,

est polycondensé avec au moins un acide di- ou polycarboxylique, un dérivé réactif d'un tel acide di-ou polycarboxylique ou au moins un di- ou polyisocyanate.

9. Utilisation d'un polycondensat selon l'une quelconque des revendications 1 à 7 dans des pièces moulées, des revêtements, des mousses, des films, des feuilles et/ou des fibres.

10. Corps moulés, revêtements, feuilles, mousses et/ou fibres, contenant un ou plusieurs polycondensats selon l'une quelconque des revendications 1 à 7.

11. Procédé de fabrication d'un ester d'acide dicarboxylique de formule (IIa)

$$RO\text{-}C(=O)\text{-}(CH_2)_{2n}\text{-}C(=O)\text{-}OR \qquad \text{(IIa)}$$

dans laquelle

R identiques ou différents, signifient alkyle en $C_1$-$C_{10}$; et
n signifie un nombre entier ≥ 12,

comprenant les étapes suivantes :

i) la mise en réaction d'un acide carboxylique de formule (IV)

$$HO\text{-}C(=O)\text{-}(CH_2)_{n-1}\text{-}CH{=}CH\text{-}R^1 \qquad \text{(IV)}$$

dans laquelle

$R^1$ signifie alkyle en $C_1$-$C_{16}$, alcényle en $C_2$-$C_{16}$, alcadiényle en $C_4$-$C_{16}$ ou alcapolyényle; et
n a la même signification que dans la formule (IIa),

en présence d'un complexe de ruthénium-carbène, qui est un composé de formule (IXa)

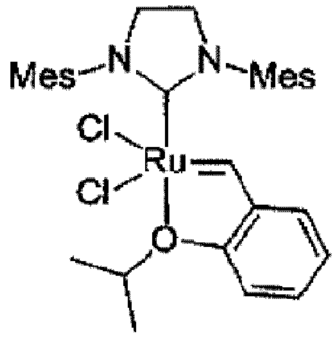

(IXa)

ou le [1,3-bis(2,4,6-triméthylphényl)-2-imidazolidinylidène]dichloro-(3-phényl-1H-indén-1-ylidène)(pyridyl)ruthénium (II),
pour former un acide dicarboxylique de formule (V)

$$HO-C(=O)-(CH_2)_{n-1}-CH=CH-(CH_2)_{n-1}-C(=O)-OH \qquad (V)$$

dans laquelle
n a la même signification que dans la formule (IIa);
ii) la mise en réaction de l'acide dicarboxylique de formule (V)
avec un ou plusieurs alcools de formule (VI)

$$ROH \qquad (VI)$$

dans laquelle R a la ou les mêmes significations que dans la formule (IIa),
en présence d'un acide
pour former un ester d'acide dicarboxylique de formule (VII)

$$RO-C(=O)-(CH_2)_{n-1}-CH=CH-(CH_2)_{n-1}-C(=O)-OR \qquad (VII)$$

dans laquelle R et n ont les mêmes significations que dans la formule (IIa) ;
iii) la mise en réaction de l'ester d'acide dicarboxylique de formule (VII)
avec un réducteur
pour former l'ester d'acide dicarboxylique de formule (IIa).

**12.** Procédé de fabrication d'un diol de formule (IIIa)

$$HO-CH_2-(CH_2)_{2n}-CH_2-OH \qquad (IIIa)$$

dans laquelle
n signifie un nombre entier $\geq$ 12,
comprenant les étapes suivantes :

i), ii), iii) la fabrication d'un ester d'acide dicarboxylique de formule (IIa) selon la revendication 11

$$RO-C(=O)-(CH_2)_{2n}-C(=O)-OR \qquad (IIa)$$

dans laquelle
n a la même signification que dans la formule (IIIa) ;

iv) la mise en réaction de l'ester d'acide dicarboxylique de formule (IIa) avec un réducteur pour former le diol de formule (IIIa).

**13.** Procédé selon la revendication 11 ou 12, dans lequel R signifie $CH_3$; n signifie 12, et $R^1$ signifie n-octyle.

**FIG. 1**

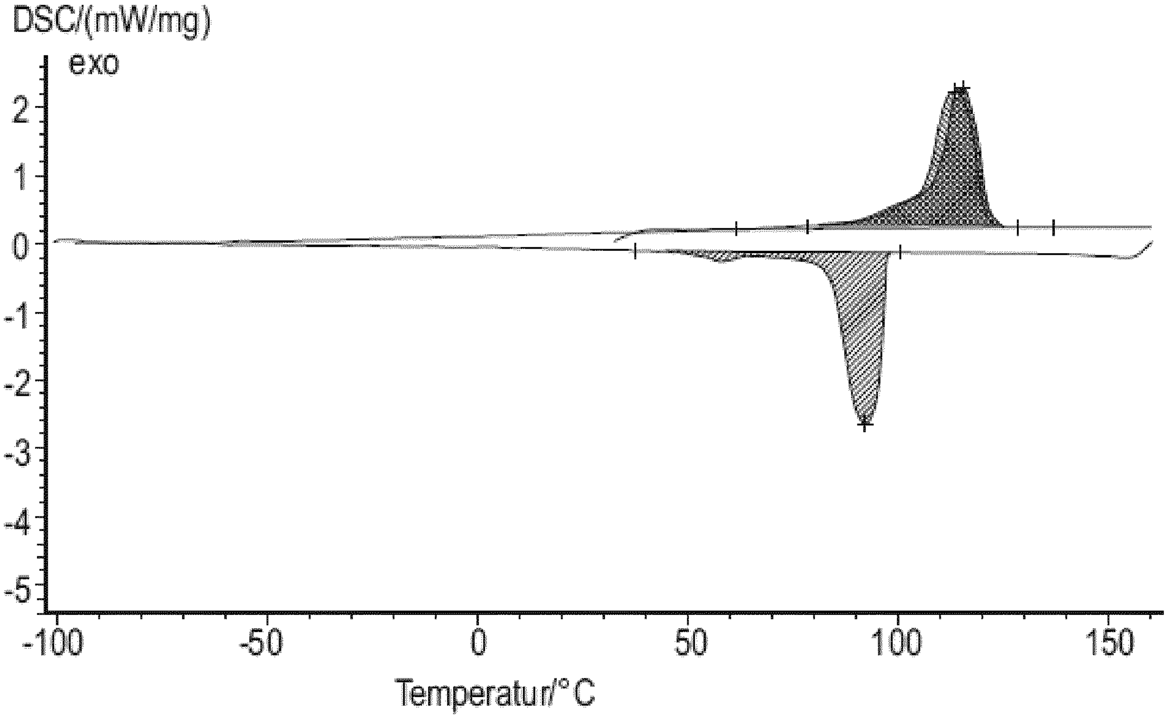

FIG. 2

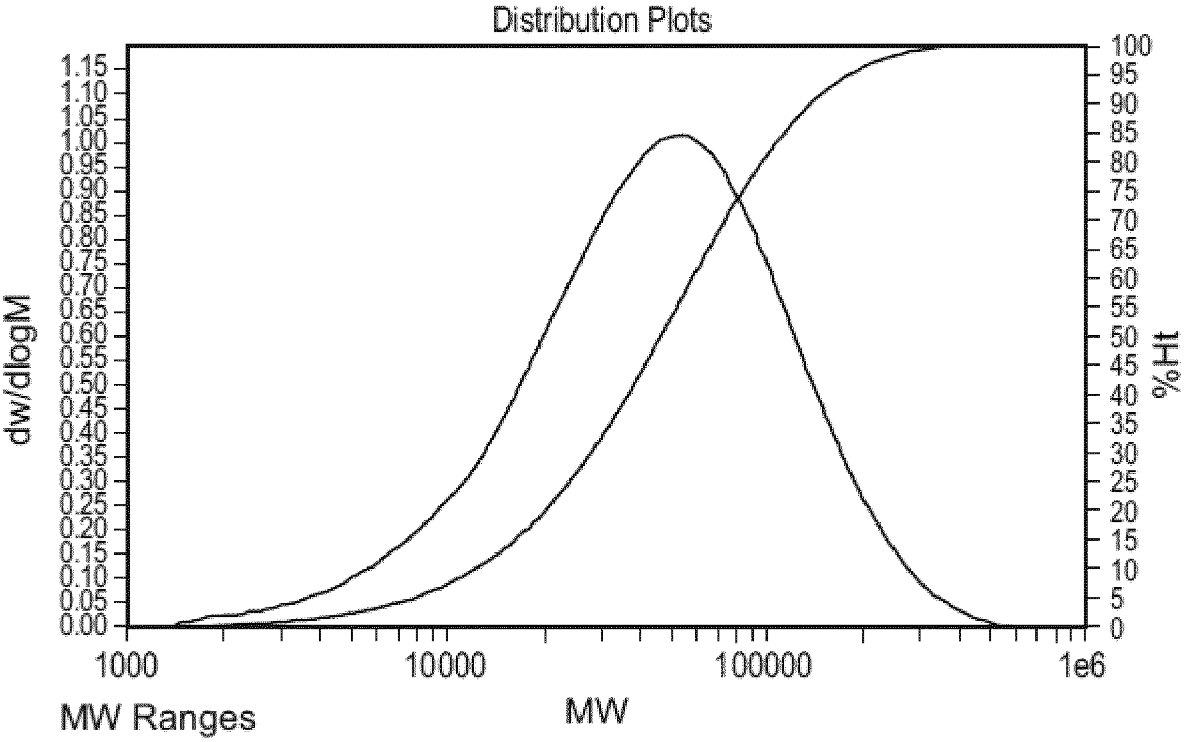
Distribution Plots
dw/dlogM
%Ht
1.15
1.10
1.05
1.00
0.95
0.90
0.85
0.80
0.75
0.70
0.65
0.60
0.55
0.50
0.45
0.40
0.35
0.30
0.25
0.20
0.15
0.10
0.05
0.00
100
95
90
85
80
75
70
65
60
55
50
45
40
35
30
25
20
15
10
5
0
1000
10000
100000
1e6
MW Ranges
MW

FIG. 3

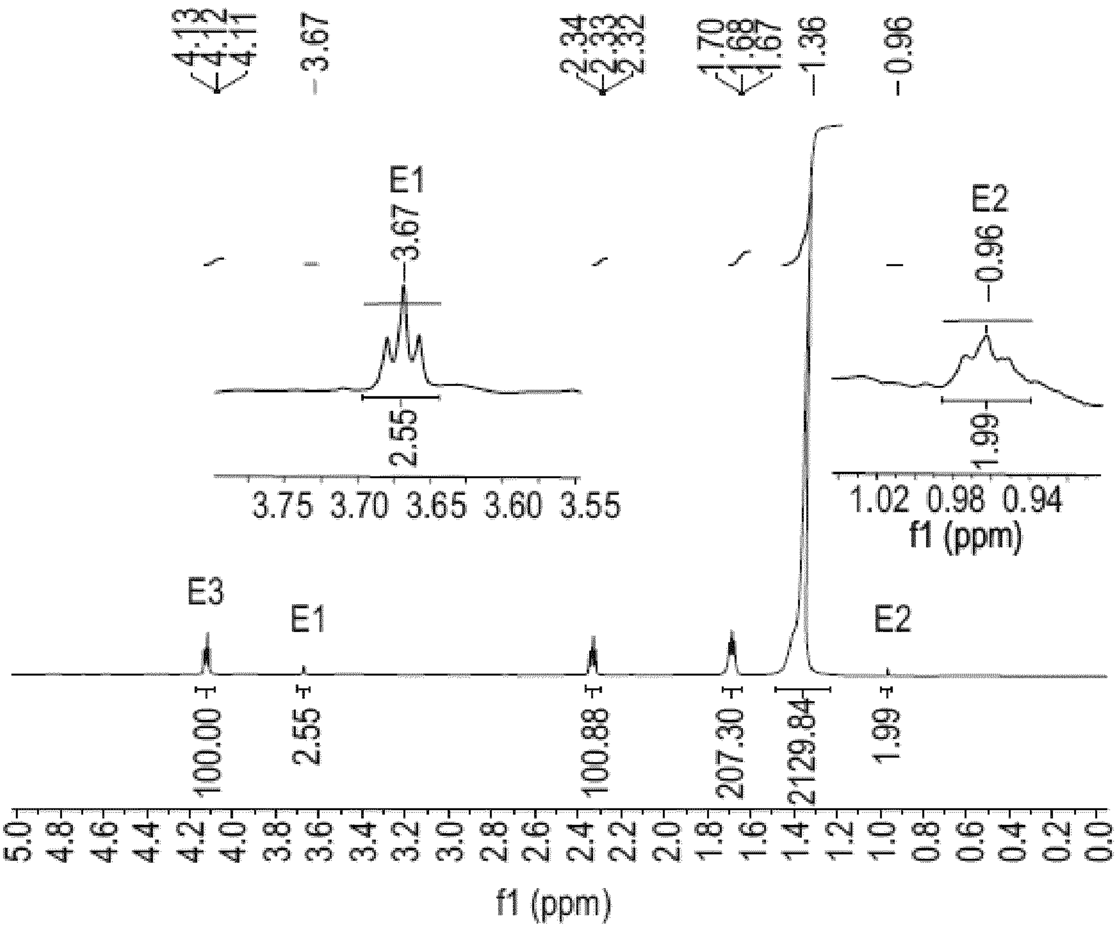
4.13
4.12
4.11
3.67
2.34
2.33
2.32
1.70
1.68
1.67
1.67
1.36
0.96
E1
3.67
2.55
3.75 3.70 3.65 3.60 3.55
E2
0.96
1.99
1.02 0.98 0.94
f1 (ppm)
E3
E1
E2
100.00
2.55
100.88
207.30
2129.84
1.99
5.0 4.8 4.6 4.4 4.2 4.0 3.8 3.6 3.4 3.2 3.0 2.8 2.6 2.4 2.2 2.0 1.8 1.6 1.4 1.2 1.0 0.8 0.6 0.4 0.2 0.0
f1 (ppm)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2011089256 A
- US 7534917 B1
- WO 2007010453 A
- WO 0015339 A
- WO 03062253 A
- WO 2008034552 A


### In der Beschreibung aufgeführte Nicht-Patentliteratur


- *Angew. Chem.,* 2000, vol. 112, 2292
- *Macromol. Chem. Phys.,* 2001, vol. 202, 1114
- *Journal of Polymer Science: Part A: Polymer Chemistry,* 2001, vol. 39, 1601
- **VAN DAM, P. B. ; MITTELMEIJER, M. C. ; BOELHOUWER, C.** *J. Chem. Soc., Chem. Commun.,* 1972, vol. 22, 1221-1222
- **VAN DAM, P. ; MITTELMEIJER, M. ; BOELHOUWER, C.** *J. Am. Oil Chem. Soc.,* 1974, vol. 51, 389-392
- **VAN DAM, P. B. ; MITTELMEIJER, M. C. ; BOELHOUWER, C.** *Fette, Seifen, Anstrichmittel,* 1974, vol. 76, 264-266
- **BUCHOWICZ, W. ; MOL, J. C.** *J. Mol. Catal. A: Chem.,* 1999, vol. 148, 97-103
- **WARWEL, S. ; DEMES, C. ; STEINKE, G.** *J. Polym. Sci., Part A: Polym. Chem.,* 2001, vol. 39, 1601-1609
- **ZAWADZKI, J.** *Skupinski React. Kinet. Catal. Lett.,* 2001, vol. 74 (1), 51-56
- **NGO, H. ; JONES, K. ; FOGLIA, T.** *J. Am. Oil Chem. Soc.,* 2006, vol. 83, 629-634
- **WARWEL, S. ; JÄGERS, H. G. ; THOMAS, S.** *Lipid/Fett,* 1992, vol. 94, 323-328
- **WARWEL, S. ; BRÜSE, F. ; DEMES, C. ; KUNZ, M. ; KLAAS, M. R. G.** *Chemosphere,* 2001, vol. 43, 39-48
- **GÜNTHARD, H. H. ; HEINEMANN, S. D. ; PRELOG, V.** *Helv. Chim. Acta,* 1953, vol. 36, 1147-1159
- **HÜNIG, S. ; LÜCKE, E. ; BRENNINGER, W.** *Org. Synt.,* 1973, vol. 5, 533
- *ORG. SYNT,* 1963, vol. 43, 34
- **ASHIKAGA, K. ; ITO, S. ; YAMAMOTO, M. ; NISHIJIMA, Y.** *J. Am. Chem. Soc.,* 1988, vol. 110, 198-204
- **DRESCHER, S. ; MEISTER, A. ; BLUME, A. ; KARLSSON, G. ; ALMGREN, M. ; DOBNER, B.** *Chem. Eur. J.,* 2007, vol. 13 (18), 5300-5307
- **GRUBBS, R.H.** Handbook of Metathesis. Wiley-VCH, Verlag GmbH & Co. KGaA, 2003
- **SANFILIPPO, D. ; RYLANDER, P. N.** Ullmann's Encyclopedia of Industrial Chemistry. Wiley-VCH Verlag GmbH & Co. KGaA, 2000
- *Angew. Chem. Int. Ed.,* 2007, vol. 46, 7473